(19) **Europäisches Patentamt**

**European Patent Office**

**Office européen des brevets**

(11) **EP 1 148 899 B1**

(12) **EUROPEAN PATENT SPECIFICATION**

(45) Date of publication and mention
of the grant of the patent:
**05.04.2006 Bulletin 2006/14**

(21) Application number: **00913281.2**

(22) Date of filing: **24.01.2000**

(51) Int Cl.:
**A61L 29/04** (2006.01)

(86) International application number:
**PCT/US2000/002191**

(87) International publication number:
**WO 2000/043051 (27.07.2000 Gazette 2000/30)**

(54) **EXPANDABLE FLUOROPOLYMER DEVICE FOR DELIVERY OF THERAPEUTIC AGENTS**

AUFWEITBARE VORRICHTUNG AUS FLUORPOLYMEREN ZUR VERABREICHUNG THERAPEUTISCHER WIRKSTOFFE

DISPOSITIF FLUOROPOLYMERE EXTENSIBLE PERMETTANT D'ADMINISTRER DES AGENTS THERAPEUTIQUES

(84) Designated Contracting States:
**AT BE CH CY DE DK ES FI FR GB GR IE IT LI LU MC NL PT SE**

(30) Priority: **25.01.1999 US 117152 P**
**01.10.1999 US 410329**
**01.10.1999 US 411797**

(43) Date of publication of application:
**31.10.2001 Bulletin 2001/44**

(73) Proprietor: **Atrium Medical Corporation**
**Hudson, NH 03051 (US)**

(72) Inventors:
• **HERWECK, Steve, A.**
**Nashua, NH 03062 (US)**
• **GINGRAS, Peter, H.**
**Windham, NH 03087 (US)**
• **MARTAKOS, Paul**
**Pelham, NH 03076 (US)**
• **KARWOSKI, Theodore**
**Hollis, NH 03049 (US)**

(74) Representative: **Frohwitter, Bernhard**
**Patent- und Rechtsanwälte,**
**Possartstrasse 20**
**81679 München (DE)**

(56) References cited:
**EP-A- 0 293 090**    **EP-A- 0 531 117**
**WO-A-97/31590**    **WO-A-98/26731**
**WO-A-98/33638**    **US-A- 4 229 838**

**Description**

**Background of the Invention**

Catheter delivered inflatable balloons are utilized in a wide range of surgical procedures to dilate, obstruct, or restore patency to body vessels and organs, as well as to maintain the position of catheter delivered instruments in vivo. Such balloons are typically attached to the distal tip of a small diameter catheter tube to facilitate delivery of the balloon to a treatment site within the body. The balloon is advanced by the catheter through a body vessel while in a deflated condition until the balloon is appropriately positioned proximate the treatment site. The balloon is inflated by infusing a fluid, such as saline, a contrast media, or water, into the balloon through an inflation lumen provided in the catheter. The inflated balloon is deflated after treatment and subsequently removed from the body.

Catheter delivered inflatable balloons can be constructed of elastomeric materials such as latex. A number of general problems are associated with such elastomeric balloons. Balloons and other expansion devices constructed of elastomeric materials can lack a maximum inflation or expansion diameter in that the prolonged application of an inflation medium will cause the balloon to continuously expand until the balloon bursts. Thus, over inflation of an elastomeric balloon may result in damage to the body vessel or organ being treated or may result in the balloon bursting within the body. Elastomeric balloons frequently do not inflate symmetrically and may not inflate to the desired size and shape. Asymmetrical expansion, as well as failure of the balloon to properly inflate, can lead to incomplete treatment of the body vessel. The high coefficient of friction of most elastomeric materials, such as latex, can result in damage to one or more cellular layers of the wall of the body vessel or organ being treated. Additionally, elastomeric expansion devices generally have insufficient strength for a number of applications, such as compressing deposits formed on vascular walls and positioning catheter delivered medical devices.

Perforated and porous catheter balloons have been proposed to deliver therapeutic agents, e.g. drugs and other medicinal agents, directly to the treatment site while concomitantly performing their primary function of dilation, obstruction, etc. The localized delivery of therapeutic agents to the treatment site can increase the effectiveness of the therapeutic agent and minimize or even negate the systemic side effects of the agent. Generally, the therapeutic agent is delivered through the wall of the catheter balloon either through openings mechanically formed in the wall or through pores present in the material used to form the balloon. A problem common to such conventional catheter balloons is that the flow-rate and the uniform delivery of fluid, and hence the therapeutic agent, through the walls of the balloon is difficult to control. Successful delivery of the therapeutic agent to the treatment site requires the therapeutic agent to penetrate the body tissue at the treatment site to a depth sufficient for the agent to effect the treatment site without effecting healthy tissue or organs proximate the tissue site. For this reason, the flow rate of and the uniform delivery of the therapeutic agent through the walls of the balloon is important. If the flow rate is too low, the therapeutic agent can fail to properly penetrate the tissue at treatment site. If the flow rate is too high, the therapeutic agent can be delivered to areas of the body outside of the treatment area and, in some cases, elevated flow rates can result in the formation of high velocity fluid jets which can traumatize the tissue adjacent the walls of the balloon.

**Summary of the Invention**

The present invention provides a radially expandable device, such as a catheter delivered inflatable balloon, having a balloon constructed a fluoropolymer material, such as expanded polytetrafluoroethylene (ePTFE). The use of fluoropolymer materials provides a radial expandable device having a biocompatible and inelastic construction that is suitable for numerous uses including the treatment of body vessels, organs, and implanted grafts. The balloon of the radially expandable device has a longitudinal axis and a wall having a thickness transverse to the longitudinal axis. The wall of the balloon is characterized by a microstructure of nodes interconnected by fibrils. The balloon of the radially expandable device is deployable from a reduced diameter, collapsed configuration to an increased diameter, expanded configuration upon application of an expansion force to the radially expandable device. Along at least a portion of the balloon substantially all the nodes of the microstructure are oriented generally perpendicularly to the longitudinal axis of the balloon. This orientation of the nodes, perpendicular to the longitudinal axis of the balloon yields a radially expandable device that predictably and dependably expands to the increased diameter configuration.

According to one aspect of the present invention, the balloon of the radially expandable device has a monolithic construction. The term "monolithic", as used herein, includes structures having a singular, unitary construction of generally homogenous material. The monolithic balloon of the radially expandable device of the present invention is characterized by a seamless construction of fluoropolymer material, such as expanded polytetrafluoroethylene (ePTFE), preferably constructed through an extrusion and expansion process. Because the cross section of the monolithic balloon is singular or unitary, the expandable device lacks seams or internal interfaces between adjacent layers that can result in unreliable expansion of the device. The monolithic construction of the balloon of the present invention contributes to the dependable and predictable expansion of the balloon to predefined, fixed maximum diameter that is generally independent of the expansion force used to radially expand the device.

A radially expandable device constructed in accordance with the method of the present invention can be dependably and predictably expanded to the second diameter upon the application of a radially deployment force within the tube. The second diameter can be predefined and fixed to a maximum expansion diameter through the manufacturing process of the present invention, resulting in an expansion device having a maximum expansion diameter that is generally independent of the radial deployment force applied to the device.

The fluoropolymer tube can be constructed through an extrusion and expansion process including the step of creating a billet by blending a mixture of a fluoropolymer and a lubricant and compressing the mixture. The fluoropolymer is preferably PTFE. The billet can then be extruded to form an extruded article. The lubricant is removed and the extruded article is expanded to form a monolithic tube of inelastic, expanded fluoropolymer material. The stretched tube is then heat set to lock in the microstructure of the tube and maintain the tube in the stretched state.

The extruded article is preferably bilaterally stretched in two opposing directions along the longitudinal axis of the article. Bilaterally stretching the extruded article yields an article that is substantially uniformly stretched over a major portion of its length and has a microstructure of nodes interconnected by fibrils. The bilateral stretching step can be carried out by displacing the ends of the extruded article either simultaneously or sequentially. The longitudinal stretch ratio of the expanded tube, i.e., the ratio of the final stretched length of the tube to the initial length, and the diametric stretch ratio, i.e., the ratio of the final diameter, after longitudinal stretching, and the initial diameter, can be varied to yield an expansion device having differing radial expansion properties. For example, the magnitude of the deployment force necessary to expand the expansion device of the present invention can be pre-selected and manipulated by varying the stretch ratios of the fluoropolymer tube. Additionally, the stretch rate can be varied to selectively provide the expansion device with improved expansion characteristics.

In accordance with another aspect of the present invention, the step of applying a radial expansion force to the fluoropolymer tube is carried out by inserting a balloon into the tube and expanding the balloon to apply the radial expansion force to the tube. Preferably, the balloon is constructed from an inelastic material such as, for example, polyethylene terephthalate (PET) or nylon. In a preferred embodiment, the balloon is constructed to be expandable to a predefined size and shape by inflation with a fluid. Radial expansion of the fluoropolymer tube with such an inelastic balloon imparts the predetermined size and shape of the balloon to the expanded fluoropolymer balloon.

In accordance with a further aspect of the present invention the step of radially expanding the fluoropolymer tube plastically deforms the tube beyond its elastic limit to the second diameter. Plastically deforming the fluoropolymer tube to the second diameter contributes to expansion device dependably expanding to the second diameter upon application of the radial deployment force.

The step of radially expanding the fluoropolymer tube can also include the steps of positioning the tube within the internal cavity of a mold fixture and radially expanding the balloon within the tube while the tube remains positioned in the internal mold cavity. The internal mold cavity preferably has a size and shape analogous to the predefined size and shape of the balloon. The internal cavity of the mold facilitates concentric radial expansion of the balloon and the fluoropolymer tube.

In accordance with another aspect of the present invention, the step of applying a radial expansion force to the fluoropolymer tube is carried out by inserting a second tube constructed from an extruded inelastic material, such as extruded PET, into the fluoropolymer tube and expanding the second tube to apply the radial expansion force to the tube. Preferably, the fluoropolymer tube and the second tube are heated to a temperature less than or equal to the glass transition temperature of the extruded material forming the second tube during the radial expansion step. The heating of the tubes can be accomplished by submerging the tubes into a hot water bath. Alternatively, the fluoropolymer tube can be expanded by the second tube within a heated mold.

In accordance with a further aspect of the present invention, the radially expandable device of the present invention is particularly suited for treatment of body passages occluded by disease. The expandable device can be utilized in the manner of a catheter balloon suitable for deployment within a body vessel by a catheter. Exemplary treatment applications of the present application include dilation of stenoic blood vessels in a percutaneous transluminal angioplasty procedure (PTA), removal of thrombi and emboli from obstructed blood vessels, urethra dilation to treat prostatic enlargement due to benign prostate hyperplasia (BPH) or prostatic cancer, and generally restoring patency to implanted grafts or body passages such as blood vessels, the urinary tract, the intestinal tract, the kidney ducts, or other body passages.

In alternative embodiment, the present invention provides a radially expandable fluid delivery device for delivering fluid to a treatment site within the body. The radially expandable fluid delivery device can be used, for example, as a catheter delivered balloon for the treatment of body lumens, organs, and grafts. Fluids, including therapeutic agents, can be delivered through the walls of the fluid delivery device to effect localized treatment of sites within the body. The fluid delivery device of the present invention is constructed of a biocompatible material having a microstructure that can provide a controlled, uniform, low-velocity distribution of fluid through the walls of the fluid delivery device to effectively deliver the fluid to the treatment site without damaging tissue proximate the walls of the device.

In accordance with one aspect of the present invention, the fluid delivery device comprises a member constructed of a biocompatible material. The member is defined by a wall having a thickness extending between an inner and an outer surface. The wall is characterized by a microstructure of nodes interconnected by fibrils. The member is deployable from a first, reduced diameter configuration to a second, increased diameter configuration upon the introduction of a pressurized fluid to the lumen. The member includes at least one microporous portion having a porosity sufficient for the pressurized fluid to permeate through the wall. The spaces between the nodes control the permeation of fluid through the wall of the fluid delivery device. In a preferred embodiment, substantially all of the nodes within the microporous portion are oriented such that spaces between the nodes form generally aligned micro-channels extending from the inner surface to the outer surface of the wall.

In accordance with another aspect of the present invention, the nodes within the microporous portion of the member can be oriented substantially parallel to the longitudinal axis of the member. The micro-channels are preferably sized to permit the passage of the pressurized fluid from the inner surface to the outer surface of the wall. The size of the microchannels within the microporous portion can be varied longitudinally and/or circumferentially to provide regions of increased porosity within the microporous portion. The presence of regions of differing porosity allows the volume of fluid delivered through the microporous portion of the member to vary, longitudinally and/or circumferentially, across the microporous portion. This allows the microporous portion to be specifically tailored to the size and shape of the site being treated.

Various biocompatible materials are suitable for the construction of the member. Expanded polytetrafluoroethylene (ePTFE), which is a hydrophobic, biocompatible, inelastic material having a low coefficient of friction, is the preferred material of choice.

In accordance with a further aspect of the present invention, the member can be provided with first and second microporous portions, each having a porosity sufficient for the pressurized fluid to permeate through the wall of the member. The first and second microporous portions can be spaced apart longitudinally and/or circumferentially. An impermeable or semi-permeable portion can be interposed between the first and second microporous portions. Further microporous portions and/or impermeable or semi-permeable portions can also be provided on the member. The microporous portions and the impermeable portions can be arranged in numerous alternative configurations. For example, microporous ring-shaped portions can be spaced along the longitudinal axis of the member. Alternatively, the microporous portions can be generally rectangular in shape and can be spaced apart about the circumference of the member. The provision of multiple microporous portions allows the fluid delivery device of the present invention to treat multiple sites within the body simultaneously.

In accordance with a further aspect of the present invention, at least one of the extrusion and expansion process parameters can be varied to form a microporous portion of the wall having a porosity sufficient for the pressurized fluid to permeate through the wall. For example, the lubricant density, the lubricant viscosity, the lubricant molecular weight, the amount of lubricant and/or the longitudinal stretch ratio can be selectively varied to form the microporous portion of the tube. The process parameters can also be varied to produce increased regions of porosity with the microporous region or to form multiple microporous regions.

In accordance with a further aspect of the present invention, the radially expandable fluid delivery device of the present invention is particularly suited for treatment of body passages and grafts occluded by disease. The fluid delivery device can be utilized in the manner of a catheter balloon suitable for deployment within a body vessel by a catheter. Exemplary treatment applications of the present application include dilation of stenoic blood vessels in a percutaneous transluminal angioplasty procedure (PCA), removal of thrombi and emboli from obstructed blood vessels, urethra dilation to treat prostatic enlargement due to benign prostate hyperplasia (BPH) or prostatic cancer, and generally restoring patency to body passages such as blood vessels, the urinary tract, the intestinal tract, the kidney ducts, or other body passages. Exemplary therapeutic agents that can be delivered through the fluid delivery device of the present invention include thrombolytics, antibiotics, antisense oligonucleaotides, chemotherapeutics, surfactants, diagnostic agents, steroids, vasodilators, vasoconstrictors, and embolic agents.

## Brief Description of the Drawings

These and other features and advantages of the present invention will be more fully understood by reference to the following detailed description in conjunction with the attached drawings in which like reference numerals refer to like elements through the different views. The drawings illustrate principles of the invention and, although not to scale, show relative dimensions.

FIGURE 1 is a side elevational view in cross-section of a radially expandable device according to the teachings of the present invention, illustrating the device in a first, reduced diameter configuration;

FIGURE 2 is a side elevational view in cross-section of the radially expandable device of FIGURE 1. illustrating the device in a second, increased diameter configuration;

FIGURE 3 is a schematic representation of the microstructure of a section of the wall of an expanded fluoropolymer tube used during the manufacturing process of the present invention to yield the radially expandable device of the present invention;

FIGURE 4A is a side elevational view in cross-section of an inelastic balloon positioned within an expanded fluoropolymer tube, illustrating the inelastic balloon in a deflated condition in accordance with a method of manufacturing a radially expandable device according to the teachings of the present invention;

FIGURE 4B is a side elevational view in cross-section of the inelastic balloon and the expanded fluoropolymer tube of FIGURE 4A, illustrating the inelastic balloon in an inflated condition in accordance with a method of manufacturing a radially expandable device according to the teachings of the present invention;

FIGURE 4C is a side elevational view in cross-section of the inelastic balloon and the expanded fluoropolymer tube of FIGURE 4A. illustrating the removal of the deflated inelastic balloon from the expanded fluoropolymer tube in accordance with a method of manufacturing a radially expandable device according to the teachings of the present invention;

FIGURE 5 is a side elevational view of an inelastic balloon and an expanded fluoropolymer tube positioned within the internal cavity of a mold fixture, illustrating the inelastic balloon in a inflated condition in accordance with a method of manufacturing a radially expandable device according to the teachings of the present invention;

FIGURE 6A is a flow chart illustrating the steps of manufacturing a radially expandable device according to the teachings of the present invention;

FIGURE 6B is a flow chart illustrating the steps of an alternative method of manufacturing a radially expandable device according to the teachings of the present invention;

FIGURE 7A is a side elevational view in cross section of a generally pear-shaped radially expandable device in accordance with the teaching of the present invention;

FIGURE 7B is a side elevational view in cross section of a generally hour glass shaped radially expandable device in accordance with the teaching of the present invention;

FIGURE 7C is a side elevational view in cross section of two coaxially aligned, adjacent radially expandable devices in accordance with the teaching of the present invention; and

FIGURE 8 is a side elevational view in cross section of a catheter deployed dilation balloon according to the teaching of the present invention, illustrating the dilation balloon expanded within a body vessel.

FIGURE 9 is a side elevational view in cross-section of a radially expandable fluid delivery device according to the teachings of the present invention, illustrating the device in a first, reduced diameter configuration;

FIGURE 10 is a side elevational view in cross-section of the radially expandable fluid delivery device of FIGURE 9, illustrating the device in a second, increased diameter configuration;

FIGURE 11 is a side elevational view of an alternative embodiment of the radially expandable fluid delivery device of the present invention, illustrating the device in the second, increased diameter configuration;

FIGURE 12 is a side elevational view of an alternative embodiment of a radially expandable fluid delivery device of the present invention having multiple axially-spaced microporous portions, illustrating the device in the second, increased diameter configuration;

FIGURE 13 is a cross-sectional view of an alternative embodiment of a radially expandable fluid delivery device of the present invention having an arcuate-shaped microporous portion, illustrating the device in the second, increased diameter configuration;

FIGURE 14 is a cross-sectional view of an alternative embodiment of a radially expandable fluid delivery device of the present invention having multiple circumferentially-spaced microporous portions, illustrating the device in the second, increased diameter configuration;

FIGURE 15 is a flow chart illustrating the steps of manufacturing a fluid delivery device according to the teachings of the present invention;

FIGURE 16 is a schematic representation of the microstructure of an expanded fluoropolymer tube used during the manufacturing process of the present invention to yield the fluid delivery device of FIGURE 9;

FIGURE 17A is a side elevational view in cross-section of an inelastic balloon positioned within an expanded fluoropolymer tube, illustrating the inelastic balloon in a deflated condition in accordance with a method of manufacturing a fluid delivery device according to the teachings of the present invention;

FIGURE 17B is a side elevational view in cross-section of the inelastic balloon and the expanded fluoropolymer tube of FIGURE 17A, illustrating the inelastic balloon in an inflated condition in accordance with a method of manufacturing a fluid delivery device according to the teachings of the present invention;

FIGURE 17C is a side elevational view in cross-section of the inelastic balloon and the expanded fluoropolymer tube of FIGURE 17A, illustrating the removal of the deflated inelastic balloon from the expanded fluoropolymer tube in accordance with a method of manufacturing a fluid delivery device according to the teachings of the present invention;

FIGURE 18 is a schematic of a system for inflating an inelastic balloon with an expanded fluoropolymer tube in accordance with a method of manufacturing a fluid delivery device according to the teachings of the present invention;

FIGURE 19 is a side elevational view of an inelastic balloon and an expanded fluoropolymer tube positioned within the internal cavity of a mold fixture, illustrating the inelastic balloon in an inflated condition in accordance with a method of manufacturing a fluid delivery device according to the teachings of the present invention;

FIGURE 20 is a flow chart illustrating the steps of an alternative method of manufacturing a fluid delivery device according to the teachings of the present invention;

FIGURE 21 is a side-elevational view in cross-section of a catheter deployed infusion balloon according to the teachings of the present invention, illustrating the infusion balloon expanded within a body vessel;

FIGURE 22 is a side elevational view in cross-section of a multi-layer radially expandable fluid delivery device in accordance with the present invention, illustrating the device in a second, increased diameter configuration.

FIGURE 23A is an electron micrograph of an external section of an ePTFE tube used in the manufacture of the fluid delivery device of the present invention;

FIGURE 23B is an electron micrograph of an external section of a fluid delivery device of the present invention; and

FIGURE 23C-23D are electron micrographs of an external section of the neck of the fluid delivery device of FIGURE 14B.

## Detailed Description of the Preferred Embodiments

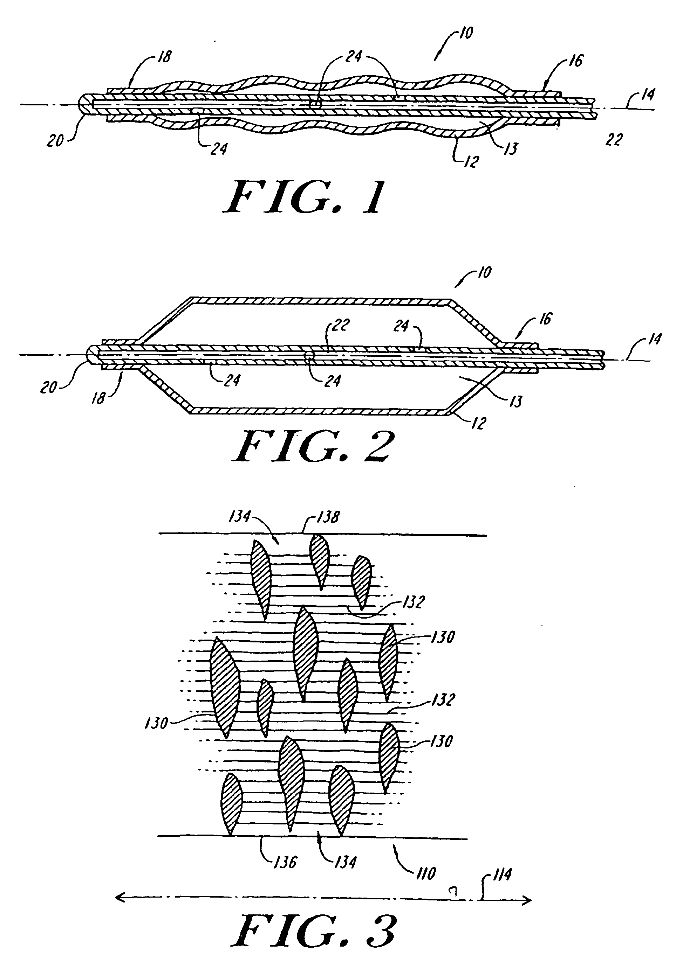

A radially expandable device 10 having a balloon 12 constructed of a generally inelastic, expanded fluoropolymer material is illustrated in FIGURES 1 and 2. Expandable devices provided by the present invention are suitable for a wide range of applications including, for example, a range of medical treatment applications. Exemplary biological applications include use as a catheter balloon for treatment of implanted grafts and body passages such as blood vessels, the urinary tract, the intestinal tract, kidney ducts, etc. Specific examples include as a device for the removal of obstructions such as emboli and thrombi from blood vessels, as a dilation device to restore patency to an occluded body passage as an occlusion device to selectively obstruct a body passage, and as a centering mechanism for transluminal instruments and catheters. The expandable device of the present invention can also be used as a sheath for covering conventional catheter balloons to control the expansion of the conventional balloon.

The balloon 12 of the radially expandable device 10 is deployable upon application of an expansion force from a first, reduced diameter configuration, illustrated in FIGURE 1, to a second, increased diameter configuration, illustrated in FIGURE 2. The balloon 12 of the expansion device 10 of the present invention preferably features a monolithic construction, i.e., the balloon 12 is a singular, unitary article of generally homogeneous material. The balloon 12 is manufactured in accordance with the methods of manufacturing of the present invention, an extrusion and expansion process described in detail below, to yield a balloon 12 characterized by a seamless construction of inelastic, expanded fluoropolymer having a predefined size and shape in the second, increased diameter configuration. The balloon 12 can be dependably and predictably expanded to the predefined, fixed maximum diameter and to the predefined shape independent of the expansion force used to expand the device.

Referring specifically to FIGURE 2, the balloon 12 of the radial expansion device 10 of the present invention is preferably generally tubular in shape when expanded, although other cross sections, such as rectangular, oval, elliptical, or polygonal, can be utilized. The cross section of the balloon 12 is preferably continuous and uniform along the length of the balloon. However, in alternative embodiments, the cross section can vary in size and/or shape along the length of the balloon. FIGURE 1 illustrates the balloon 12 relaxed in the first, reduced diameter configuration. The balloon 12 has a central lumen 13 extending along a longitudinal axis 14 between a first end 16 and second end 18.

A deployment mechanism in the form of an elongated hollow tube 20 is shown positioned within the central lumen 13 to provide a radial deployment or expansion force to the balloon 12. The radial deployment force effects radial expansion of the balloon 12 from the first configuration to the second increased diameter configuration illustrated in FIGURE 2. The first end 16 and the second end 18 are connected in sealing relationship to the outer surface of the hollow tube 20. The first and second ends 16 and 18 can be thermally bonded, bonded by means of an adhesive, or attached by other means suitable for inhibiting fluid leakage from the first and second ends 16 and 18 between the walls of the balloon 12 and the tube 20.

The hollow tube 20 includes an internal, longitudinal extending lumen 22 and a number of side-holes 24 that provide for fluid communication between the exterior of the tube 20 and the lumen 22. The tube 20 can be coupled to a fluid source (not shown) to selectively provide fluid, such as water, saline, or air, to the lumen 13 of the balloon 12 through the lumen 22 and side-holes 24. The pressure from the fluid provides a radial expansion force on the balloon 12 to radial expand the balloon 12 to the second, increased diameter configuration. Because the balloon 12 is constructed from an inelastic material, uncoupling the tube 20 from the fluid source or otherwise substantially reducing the fluid pressure within the lumen 13 of the balloon 12, does not generally result in the balloon 12 returning to the first, reduced diameter configuration. However, the balloon 12 will collapse under its own weight to a reduced diameter. Application of negative pressure. from, for example, a vacuum source, can be used to completely deflate the balloon 12 to the initial reduced diameter configuration.

One skilled in the art will appreciate that the expansion device 10 of the present invention is not limited to use with deployment mechanisms employing a fluid deployment force, such as hollow tube 20. Other known deployment mechanisms can be used to radially deploy the expansion device 10 including, for example, mechanical operated expansion elements, such as mechanically activated members or mechanical elements constructed from temperature activated materials such as nitinol.

Various fluoropolymer materials are suitable for use in the present invention. Suitable fluoropolymer materials include, for example, polytetrafluoroethylene (PTFE) or copolymers of tetrafluoroethylene with other monomers may be used. Such monomers include ethylene, chlorotrifluoroethylene, perfluoroalkoxytetrafluoroethylene, or fluorinated propylenes such as hexafluoropropylene. PTFE is the preferred material of choice. Accordingly, while the radial expansion device 10 can be manufactured from various fluoropolymer materials, and the manufacturing methods of the present invention can utilize various fluoropolymer materials, the description set forth herein refers specifically to PTFE.

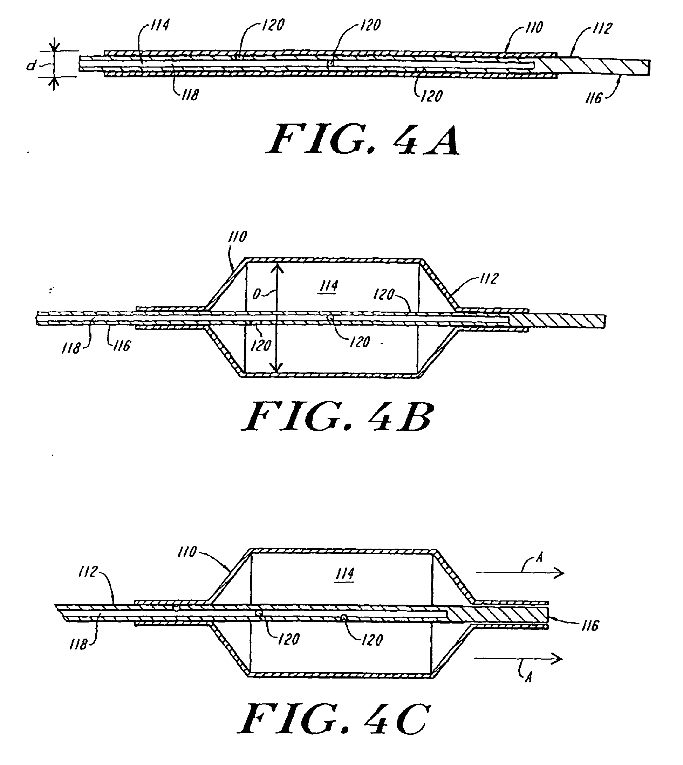

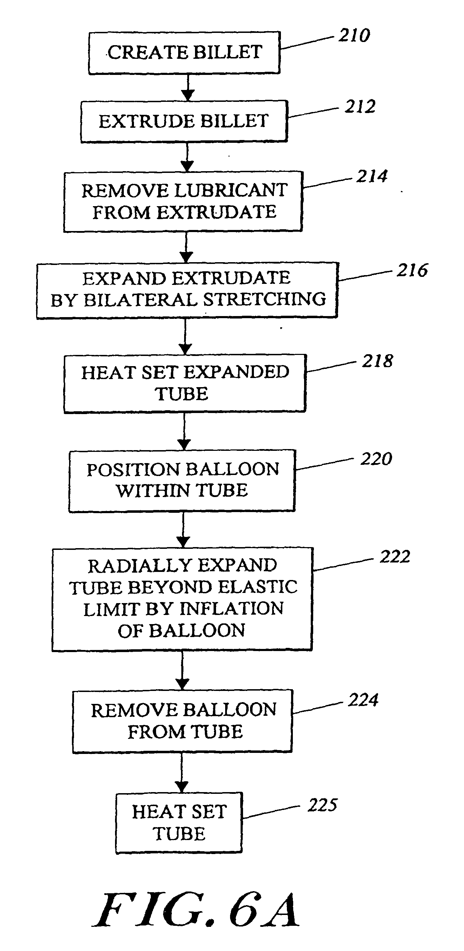

A method of manufacturing a radially expandable device in accordance with the present invention will be described in connection with FIGURES 4A-4C and the flow chart shown in FIGURE 6A. The radially expandable device 10 of the present invention is produced from a tube 110 constructed of expanded fluoropolymer material, which is preferably produced through an extrusion and a longitudinal expansion process. The preferred fluoropolymer material is expanded PTFE (ePTFE), which is a hydrophobic, biocompatible, inelastic material having a low coefficient of friction, although, as discussed above, other inelastic, biocompatible fluoropolymer materials may be used.

To produce the ePTFE tube, a billet comprising a PTFE resin mixed with an organic lubricant is utilized. Various organic lubricants are suitable such as naphtha, ISOPAR-G and ISOPAR-H available from Exxon Corporation. The blended resin is compressed at low pressure to yield a tubular billet of PTFE resin and lubricant, step 210 of FIGURE 6A. The tubular billet is then extruded through an extruder, for example a ram extruder, to reduce the cross section of the billet and to yield a tubular extrudate, step 212. The organic lubricant can be removed from the extrudate by drying the extrudate in a heated oven, step 214.

Once the tubular extrudate is produced, the extrudate is expanded by longitudinal stretching, step 216. Preferably, the extrudate is bilaterally stretched. Bilateral stretching is accomplished by displacing both ends of the extrudate, sequentially or simultaneously, away from the center of the extrudate. Bilateral stretching provides a material that is homogeneously stretched over the majority of its length. After the extrudate has been stretched, it is heat set to lock in the microstructure of the material, step 218 of FIGURE 6A, and to complete the process of the forming the tube 110 of ePTFE.

FIGURE 3 is a schematic representation of the microstructure of the walls of the ePTFE tube 110 as formed by the extrusion and expansion process described above. For purposes of description, the microstructure of the tube 110 has been exaggerated. Accordingly, while the dimensions of the microstructure are enlarged, the general character of the illustrated microstructure is representative of the microstructure prevailing within the tube 110.

The microstructure of the ePTFE tube 110 is characterized by nodes 130 interconnected by fibrils 132. The nodes 130 are generally oriented perpendicular to the longitudinal axis 114 of the tube 110. This microstructure of nodes 130 interconnected by fibrils 132 provides a microporous structure having microfibrillar spaces which define through-pores or channels 134 extending entirely from the inner wall 136 and the outer wall 138 of the tube 110. The through-pores 134 are perpendicularly oriented (relative to the longitudinal axis 114), internodal spaces that traverse from the inner wall 136 to the outer wall 138. The size and geometry of the through-pores 134 can be altered through the extrusion and stretching process, as described in detail below, to yield a microstructure that is impermeable, semi-impermeable, or permeable.

In a preferred embodiment, the ePTFE tube 110, and the resultant expandable device 10, has a fine nodal structure that is uniform throughout the cross section and length of the ePTFE tube. The preferred uniform fine nodal structure provides the expandable device 10 with improved expansion characteristics as the expandable device dependably and predictably expands to the second diameter. The preferred fine nodal structure is characterized by nodes having a size and mass less than the nodes found in conventional ePTFE grafts, preferably in the range of $25\mu m$ - $30 \mu m$. Additionally, the spacing between the nodes, referred to as the internodal distance, and the spacing between the fibers, referred to as the interfibril distance, is also preferably less than found in conventional ePTFE grafts, preferably in the range of $1\mu m$ - $5 \mu m$. Moreover, the internodal distance and the interfibril distance in the preferred embodiment is preferably uniform throughout the length and the cross section of the ePTFE tube. The preferred uniform nodal structure can be created by forming the billet with a uniform lubricant level throughout its cross section and length. Stretching the tubular extrudate at higher stretch rates, for example at rates greater than 1 in/s, yields the preferred fine nodal structure. Preferably, the extrudate is stretched at a rate of approximately 10 in/s or greater.

Continuing to describe the manufacturing method of the present invention and referring again to FIGURES 4A and 6A, the ePTFE tube 110, having an initial diameter d, is pulled over a balloon 112 to position the balloon 112 within the lumen 114 of the tube 110, step 220 of FIGURE 6A. The balloon 112 is preferably constructed of an inelastic material such as, for example, PET or nylon, such that the balloon 112, when inflated, attains a predetermined size and shape. The balloon 112 can be bonded or otherwise coupled to a rigid catheter or hypo-tube 116 to facilitate placement and removal of the ePTFE tube as described below. The catheter 116 has a central inflation lumen 118 and a plurality of side-holes 120 to provide for the delivery of an inflation fluid to inflate the balloon 112.

Referring specifically to FIGRUE 4B, the balloon 112 can be inflated by introduction of a pressurized fluid to the lumen 114 of the ePTFE tube 110. The overlying ePTFE tube 110 expands with the inelastic balloon 122 until both the balloon 112 and the ePTFE tube 110 obtain the predetermined size and shape of the inflated balloon 112, step 222 of FIGURE 6A. The inflated balloon 112 thus imparts its predetermined size and shape to the ePTFE tube 110. This radially expansion process is referred to as blow-molding. The PTFE tube 110 shown in FIGURE 4B is radially expanded from the initial diameter d (FIGURE 4A) to an increased diameter D. This radial expansion process may take place in an air, water, or steam-heated chamber that is heated to a temperature between 35°C and 60°C, preferably 50°C. The elevated temperature can contribute to uniform expansion, both circumferentially and longitudinally, of the ePTFE balloon, as well as uniform wall thickness.

It is preferable for the ePTFE tube 110 to be plastically deformed by the radial expansion of the inelastic balloon 112, step 222 of FIGURE 6A. The terms "plastic deformation" and "plastically deform," as used herein, is intended to include the radial expansion of the ePTFE tube 110 beyond the elastic limit of the ePTFE material such that the ePTFE material is permanently deformed. Once plastically deformed, the ePTFE material forming the tube 110 becomes substantially inelastic, i.e., the ePTFE tube generally will not, on its own, return to its pre-expansion size and shape.

The ePTFE tube 110 can be removed from the balloon 112 by sliding the ePTFE tube 110 relative to balloon 112 and catheter 116, i.e. in the direction of arrows A in FIGURE 4C, step 224 of FIGURE 6A. The tube 110 can be heat set at a temperature above the sinter point of the material forming the tube, 360°C for ePTFE, to lock in the structure of the tube 110, step 225 of FIGURE 6A.

The resultant radially expanded ePTFE tube 110, produced in accordance with the above described method, provides a radially expandable device, such as expandable device 10 illustrated in FIGURES 1 and 2 and described above, that is radially expandable from a relaxed, collapsed diameter to the second, increased diameter D upon application of a radial deployment force from a deployment mechanism, e.g., hollow tube 20, within the tube 110. The ePTFE tube 110 further provides an expansion device 10 having monolithic construction, that is, a singular, unitary construction of generally homogenous material, ePTFE, that lacks seams or other internal interfaces. The ePTFE tube 110 can be dependably and predictably expanded to the second diameter D upon the application of the radially deployment force within the tube. In particular, the plastically deformed, monolithic microstructure of the ePTFE tube 110, once radially expanded by the inelastic balloon 120, will readily return to the increased diameter D upon application of a radial deployment force and generally will not expand beyond the increased diameter D. The increased diameter D is effectively the maximum expansion diameter for the ePTFE tube, as the increased diameter D is generally independent of the radial deployment force applied to the tube.

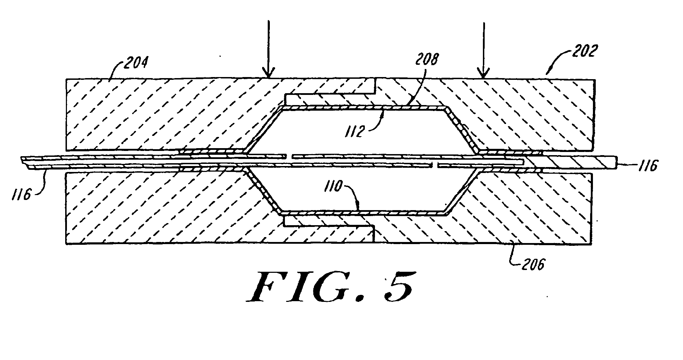

Referring to FIGURE 5, an alternative method of manufacturing a radially expandable device employing a mold 202 is illustrated. The mold 202 includes two interconnected sections 204 and 206 forming an internal mold cavity 208 for receiving the ePTFE tube 110 with the balloon 112 positioned therein. The mold 202 is preferably constructed of a rigid, unyielding material such as a metal or metal alloy. Suitable metals or metal alloys include brass and steel alloys. The internal mold cavity 208 preferably has a size and shape analogous to that of the inflated balloon 112 to ensure that the inflated balloon 112, and the overlying ePTFE tube 110 concentrically expand.

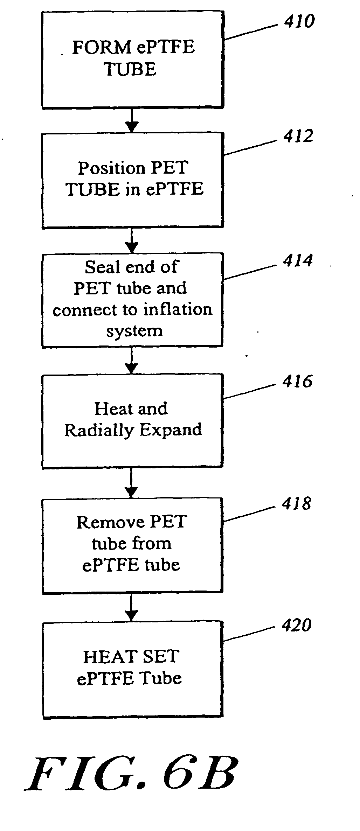

Referring to the flow chart illustrated in FIGURE 6B, a further alternative method of manufacturing a radially expandable device according to the teachings of the present invention will be described. A tube constructed of ePTFE is formed in accordance with the methods described above, step 410. A tube formed of an extruded inelastic material such as PET is used in place of balloon 112 to radially expand the ePTFE tube. The extruded tube is positioned within the ePTFE tube 110, step 412. The extruded tube is then sealed at one end and attached to an inflation system at the other end, step 414. The extruded tube can then be inflated by an inflation medium to radially expand the ePTFE tube, step 416. The extruded tube and ePTFE tube are preferably heated to the glass transition temperature of the extruded tube, approximately 80°C - 100°C for PET, as the extruded tube is inflated within the ePTFE tube. It is preferable to limit the temperature of the extruded tube to a temperature less than or equal to the glass transition temperature of the material forming the extruded tube to facilitate removal of the extruded tube from the ePTFE tube. Heating the extruded tube to a temperature above the glass transition temperature will cause the extruded tube to heat set in an expanded configuration, which makes removing the extruded tube from the ePTFE tube difficult. A suitable inflation system employing a hot water chamber for heating the tubes is described below.

After the extruded tube and ePTFE tube are expanded to desired size and shape, the extruded tube is deflated and removed from the ePTFE tube, step 418. The ePTFE tube is then heat set to lock in the structure of the ePTFE tube, step 420.

A mold, such as mold 202, can be employed during radial expansion of the ePTFE tube using the PET tube. The mold is preferably heated within the hot water chamber of the inflation system or by other means such as a hot oil bath or through a steam, hot air, electric, radio frequency or infra red heat source. The mold can be constructed of a material having good heat transfer characteristics, such as metal or metal alloy, for example brass. The mold includes a mold cavity having a size and shape analogous to the desired size and shape of the radially expandable device 10 in the second diameter configuration.

Expansion devices of a wide variety of sizes and shapes may be constructed by altering the geometry of the inelastic balloon 112 or the mold 202. Accordingly, an ePTFE expansion device having a size and shape tailored to a particular function can be manufactured in accordance with the manufacturing methods of the present invention by selecting an inelastic balloon having the desired size and shape. Exemplary expandable fluoropolymer medical treatment devices of different size and shapes are illustrated in FIGURES 7A-7C.

FIGURE 7A illustrates a radially expandable treatment device 10A having a generally pear-shaped configuration when inflated. The pear shaped configuration is particularly suited for removal of obstructions, such as thrombi and emboli, from a body passage. The expandable treatment device 10A has an increased diameter section 226 that tapers to a reduced diameter section 228. The diameter of the increased diameter section 226 is preferably equal to or slightly less than the diameter of the body passage. The increased diameter section 226 is the primary mechanism for removing obstructions from the body passage and, thus, preferably substantially fills the entire diameter of the body passage to facilitate complete removal of all obstructions from the body passage. The pear-shaped configuration provides the expandable treatment device 10A with a limited, reduced surface area, the increased diameter section 226, which can engage the walls of the body passage and thus minimizes potential damage to the walls of the body passage.

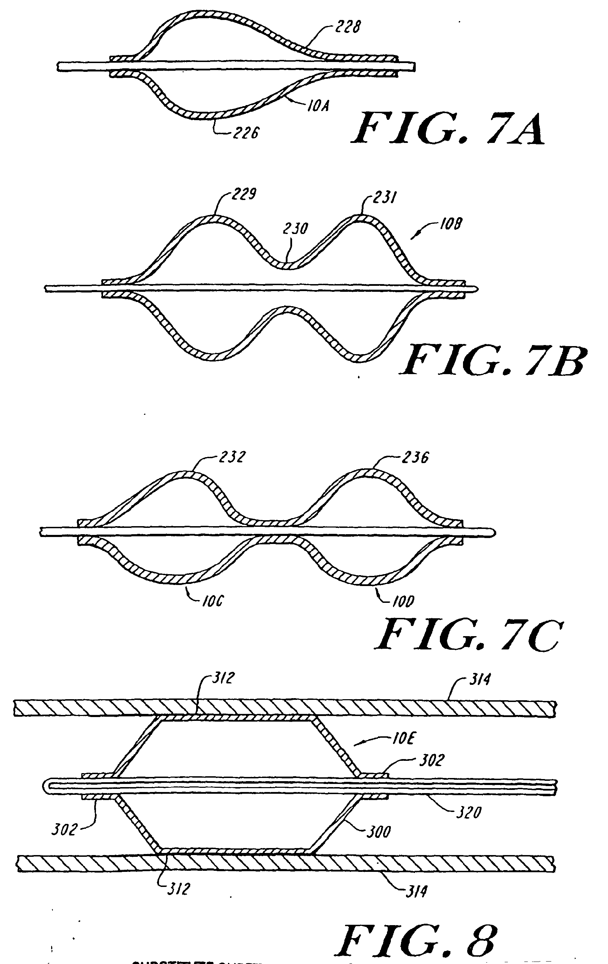

FIGURES 7B and 7C illustrate alternative exemplary embodiments of the expandable device of the present invention, each providing the device with a reduced surface area for contacting the walls of a body passage. In particular, FIGURE 7B illustrates a substantially hour-glass shaped expandable treatment device 10B including, when inflated, a first increased diameter section 229 that tapers to a reduced diameter section 230 that expands to a second increased diameter section 231. As in the case of the exemplary embodiment described above and illustrated in FIGURE 7A, the first and second increased diameter sections 229 and 231 preferably have a diameter equal to or slightly less than the diameter of a body passage to be treated to facilitate complete removal of obstructions from the body passage.

FIGURE 7C illustrates a third exemplary embodiment in which two axially aligned expandable devices 10C and 10D are provided. As is the case of the second exemplary embodiment described above, the dual expandable devices 10C and 10D together provide a substantially hour-glass configuration that provides the devices with two increased diameter sections 232 and 236.

One feature of the manufacturing processes of the present invention is that the properties of the ePTFE tube 110 forming the expandable device 10 can be manipulated, by varying the extrusion and expansion process parameters, to produce a radially expandable device 10 having different expansion characteristics. For example, the longitudinal stretch ratio of the ePTFE tube 110, i.e., the ratio of final stretched length of the tube to the initial length, and the diametric stretch ratio of the ePTFE tube 110, i.e., the ratio of the final diameter, after longitudinal stretching, and the initial diameter, and the stretch rate can be varied to yield an expansion device having different radial expansion properties. Applicants determined that larger longitudinal stretch ratios, in the order of 2:1 to 3:1, can result in a ePTFE tube having a microstructure characterized by increased internodal distances and interstitial space. Suitable longitudinal stretch ratios can be from 1.1:1 to 10: 1. As discussed above, Applicants determined that increased stretch rates yield an ePTFE tube having a fine nodal structure conducive to radial expansion. Expansion devices constructed from ePTFE tubes having such larger longitudinal and/or diametric stretch ratios and which are stretched at increased rates generally require less radial deployment force to expand from the collapsed, reduced diameter configuration to the expanded, increased diameter configuration. Thus, the magnitude of the radial deployment force necessary to expand the ePTFE tube 110 can be pre-selected and manipulated by varying the stretch ratios and stretch rate of the ePTFE tube 110 during the manufacturing process.

In addition to the longitudinal and diametric stretch ratios and the stretch rate , further process parameters can be varied to produce an ePTFE tube 110 having different characteristics. For example, the ePTFE tube 110 can be manufactured to have a porosity that allows for the fluid utilized to radially deploy the ePTFE tube to the expanded configuration to permeate through the walls of the ePTFE tube at a desired flow rate. The process for producing such a microporous ePTFE tube is described in detail in below.

FIGURE 8 illustrates an exemplary embodiment of the expandable device of the present invention in which the expandable device 10E is utilized as a catheter deployed dilation balloon 300 for the treatment of a blood vessel 310 partially occluded by plaque deposits 312 adhered to the walls 314 of the blood vessel. The dilation balloon 300 can be manufactured in accordance with the methods of the present invention and is shown in the expanded configuration. The ends 302 of the dilation balloon 300 are bonded to a catheter tube 320, which is used to provide an inflation fluid to the balloon 300 to effect expansion of the balloon 300 to a predefined and fixed maximum diameter.

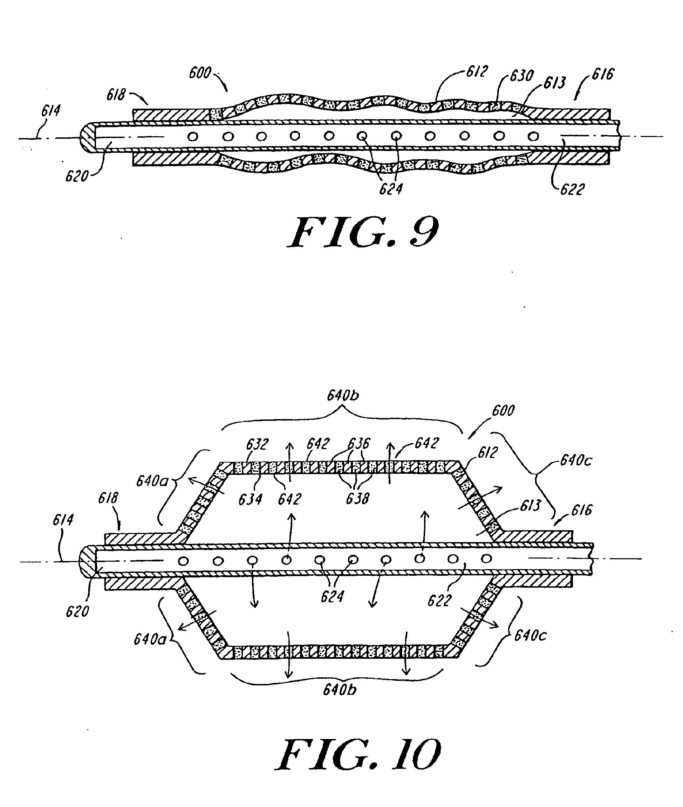

A radially expandable fluid delivery device 600 in accordance with an alternative embodiment of the present invention has an extensible member 612 constructed of a biocompatible fluoropolymer material is illustrated in FIGURES 9 and 10. The radially expandable fluid delivery device 600 of the present invention is suitable for a wide range of treatment applications. Such applications include use of the fluid delivery device 600 as a catheter balloon for treatment of body passages and grafts such as blood vessels, the urinary tract, the intestinal tract, kidney ducts, natural and synthetic grafts, etc. Specific examples include as a device for the removal of obstructions such as emboli and thrombi from blood vessels, as a dilation device to restore patency to an occluded body passage, as an occlusion device to selectively obstruct a body passage, and as a centering mechanism for transluminal instruments and catheters. In each of these applications, the fluid delivery device 600 can simultaneously deliver a fluid, such as a therapeutic agent, to the body vessel, or the tissue or organs surrounding the body vessel, to effect local treatment with the agent.

The extensible member 612 of the radially expandable fluid delivery device 600 is deployable upon application of an expansion force from a first, reduced diameter configuration, illustrated in FIGURE 9, to a second, increased diameter configuration, illustrated in FIGURE 10. The extensible member 612 of the fluid delivery device 600 of the present invention preferably features a monolithic construction, i.e., the extensible member 612 is a singular, unitary article of generally homogeneous material. The extensible member 612 is manufactured in accordance with the methods of manufacturing of the present invention, an extrusion and expansion process described in detail below, to yield an extensible member 612 characterized by a seamless construction of inelastic, expanded fluoropolymer having a predefined size and shape in the second, increased diameter configuration. The extensible member 612 can be dependably and predictably expanded to the predefined, fixed maximum diameter and to the predefined shape independent of the expansion force used to expand the device.

Referring specifically to FIGURE 10, the extensible member 612 of the fluid delivery device 600 of the present invention is generally tubular in shape when expanded, although other cross sections, such as rectangular, oval, elliptical, or polygonal, can be utilized. The cross-section of the extensible member 612 is continuous and uniform along the length of the extensible member. However, in alternative embodiments, the cross-section can vary in size and/or shape along the length of the extensible member. FIGURE 9 illustrates the extensible member 612 relaxed in the first, reduced diameter configuration. The extensible member 612 has a central lumen 613 extending along a longitudinal axis 614 between a first end 616 and second end 618.

A deployment mechanism in the form of an elongated hollow tube 620 is shown positioned within the central lumen 613 to provide a radial deployment or expansion force to the extensible member 612. The tube 620 can be, for example, a catheter constructed from PEBAX tubing available from Elf Atochem of France. The tube 620 is selected to have an outer diameter approximately equal to the diameter of the central lumen 613 of the extensible member 612 in the reduced diameter configuration. The radial deployment force effects radial expansion of the extensible member 612 from the first configuration to the second increased diameter configuration illustrated in FIGURE 10. The first end 616 and the second end 618 are connected in sealing relationship to the outer surface of the hollow tube 620. The first and second ends 616 and 618 can be thermally bonded, bonded by means of an adhesive, or attached by other means suitable for inhibiting fluid leakage from the first and second ends 616 and 618 between the walls of the extensible member 612 and the tube 620.

The hollow tube 620 includes an internal, longitudinal extending lumen 622 and a number of side-holes 624 that provide for fluid communication between the exterior of the tube 620 and the lumen 622. The tube 620 can be coupled to a fluid source (not shown) to selectively provide fluid, such as water, a contrast medium, or saline, to the lumen 613 of the extensible member 612 through the lumen 622 and side-holes 624. The pressure from the fluid provides a radial expansion force on the extensible member 612 to radial expand the extensible member 612 to the second, increased diameter configuration. Because the extensible member 612 is constructed from an inelastic material, uncoupling the tube 620 from the fluid source or otherwise substantially reducing the fluid pressure within the lumen 613 of the extensible member 612, does not generally result in the extensible member 612 returning to the first, reduced diameter configuration. However, the extensible member 612 will collapse under its own weight to a reduced diameter. Application of negative pressure, from, for example, a vacuum source, can be used to completely deflate the extensible member 612 to the initial reduced diameter configuration.

One skilled in the art will appreciate that the expansion device 600 of the present invention is not limited to use with deployment mechanisms employing a fluid deployment force, such as hollow tube 620. Other known deployment mechanisms can be used to radially deploy the expansion device 600 including, for example, mechanical operated expansion elements, such as mechanically activated members or expansion elements constructed from temperature activated materials such as nitinol.

The extensible member 612 is defined by a wall 630 of biocompatible fluoropolymer material having a thickness extending between an outer surface 632 and an inner surface 634 of the extensible member 612. The inner surface 634 defines the lumen 613 of the extensible body 612. The wall 612 of biocompatible fluoropolymer material is characterized by a microstructure of nodes interconnected by fibrils. FIGURES 9 and 10 provide schematic representations of the microstructure of the extensible member 612. For purposes of description, the microstructure of the extensible member has been exaggerated. Accordingly, while the dimensions of the microstructure are enlarged, the general character of the illustrated microstructure is representative of the microstructure of the extensible member 612.

The extensible member 612 includes at least one microporous portion 640 having a porosity sufficient for the flow of fluid from the lumen 613 through the wall 630, i.e. from inner surface 634 to outer surface 632. The microstructure of the wall 630 within the microporous portion 640 is characterized by a plurality of nodes 636 interconnected by fibrils 638. Preferably, all or substantially all of the nodes 636 within the microporous portion 640 are oriented perpendicular to the inner surface 634 and the outer surface 632 of the extensible member 612 as well as parallel to each other. This preferred orientation of the nodes 636 provides internodal spaces that form pores or micro-channels 642 that extend between the inner surface 634 and the outer surface 632 of the extensible member 612. The micro-channels 642 are sized to permit the flow of fluid between the nodes 636 through the wall 630.

The size of the micro-channels or pores 642 can be selected through the manufacturing process of the present invention, described in detail below. Preferably, the internodal distance of microstructure of the wall within the microporous region, and hence the width of the micro-channels, is approximately 1 $\mu$m to approximately 150 $\mu$m. Internodal distances of this magnitude can yield flow rates of approximately 0.01 ml/min to approximately 100 ml/min of fluid through the wall 630 of the extensible member 612.

In a preferred embodiment, the size of the micro-channels or pores 642 within the microporous portion 640 is uniform throughout the microporous portion. In this manner, the flow-rate of fluid through the wall of the microporous portion 640 is also generally uniform. In some applications, however, it may be desirable to have an area of increased or decreased porosity within the microporous portion such that the flow-rate can be tailored to the particular geometry of the site being treated. For example, if a particular region at the treatment site requires increased concentrations of the therapeutic agent, the flow rate at the corresponding section of the microporous portion can be increased to thereby increase the volume of therapeutic agent be delivered to the particular region. Additionally, it is often desirable to reduce the volume of therapeutic agent delivered to the periphery of the treatment site to minimize damage to healthy tissue adjacent the site. The pore size within the microporous region thus can be varied axially or circumferentially, or both, to form such areas of increased or decreased porosity.

The terms "axial" and "axially" as used herein refers to a direction generally parallel to the longitudinal axis of the extensible member 612. The terms "circumferential" and "circumferentially" as used herein refers to a direction generally parallel to the circumference of the extensible member 612. The explanation of these terms is not however, to be construed to limit the extensible member 612 to a circular cross-section. As discussed above, the cross-section can be any of a number shapes. In the case of non-circular cross-sections, the terms "circumferential" and "circumferentially" as used herein to generally refer to a direction parallel to the perimeter of the extensible member.

The axial length, as well as the axial position, of the microporous portion 640 of the extensible member 612 can be varied to accommodate specific treatment applications. As illustrated in FIGURE 10, for example, the microporous portion 640 can extend along the entire inflatable length of the extensible member 612. In particular, the microporous portion 640 includes a section 640b that is oriented parallel to the longitudinal axis 614 of the extensible member 612 as well as two spaced apart tapered sections 640b and 640c. In the embodiment illustrated in FIGURE 10, thus, the microporous portions 640b, 640c, and 640c extend along the entire length of the extensible member 612, absent the first and second ends 616 and 618, which are sealed to the hollow tube 612.

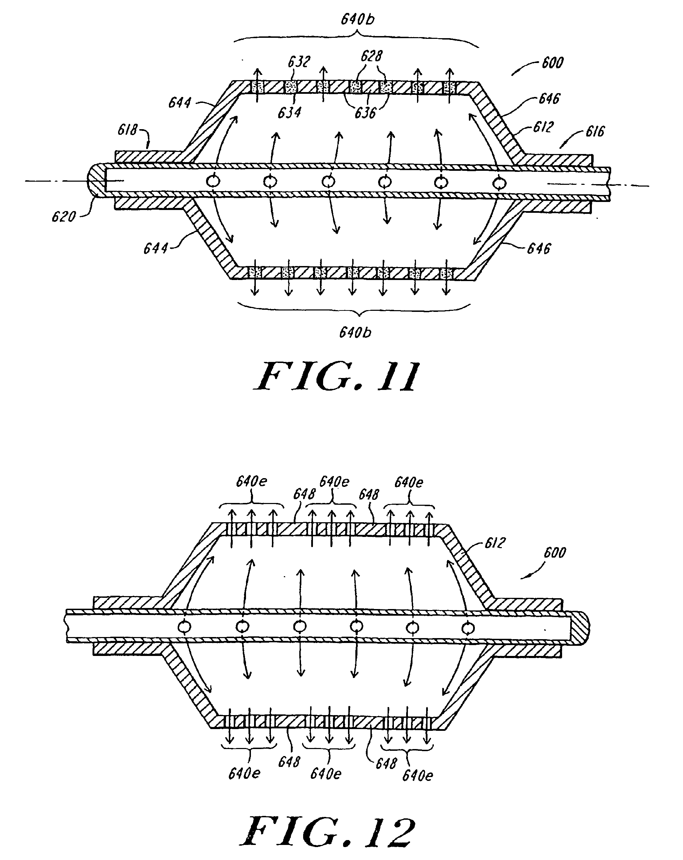

The microporous portion 640 need not extend the entire inflatable length of the extensible member 612 but instead can include only sections of the length of the extensible member 612. For example, as illustrated in FIGURE 11, the microporous portion can include only section 640b, the section oriented parallel to the longitudinal axis 614 of the extensible member 612. Tapered section 644 and 646 adjacent the microporous portion 640b can be substantially impermeable to the fluid within the extensible member 612. The tapered sections 644 and 646 can formed with the same microstructure as the microporous portions 640b, i.e. nodes of the same size and orientation, and can be sealed with a coating provided on the inner surface 634 or the outer surface 632 to inhibit fluid flow through the sections 644 and 646. Alternatively, the microstructure of the tapered sections 644 and 646 can have an impermeable, non-porous structure. For example, the nodes forming the microstructure of the tapered sections 644 and 646 can be spaced apart or oriented to inhibit or prevent fluid from passing therethrough.

Moreover, two or more microporous portions of similar or different axial lengths can be provided on the extensible member 612. For example, the extensible member 612 can be provided with three axially spaced microporous portions 640e, as shown in FIGURE 12. Each of the microporous portions 640e is bordered axially by an impermeable portion 648. The microporous portions 640e provide porous, fluid permeable annular zones that are spaced lengthwise along the extensible member 612. The microporous portions 640e can be equally spaced and of similar size, as illustrated in FIGURE 12, or, in the alternative, can be distinctly sized and spaced. One skilled in the art will appreciate that the number of independent microporous portions is not limited to three as shown in FIGURE 12, but can include two or more zones as dictated by the length of the extensible member 612 and the desired size of the microporous portions.

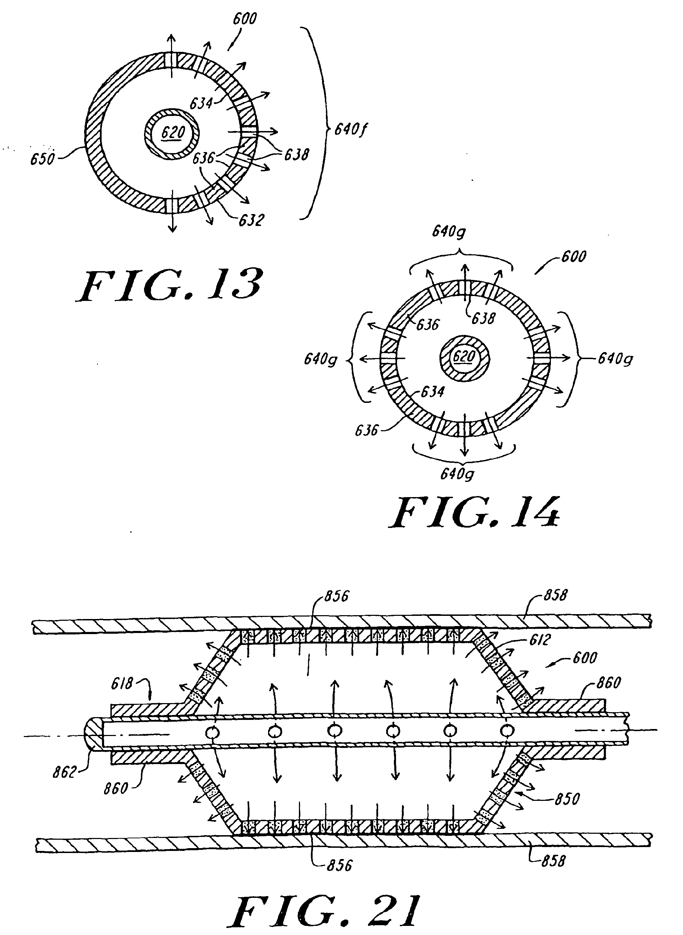

Referring to FIGURES 13 and 14, the size and position of the microporous portion can be varied circumferentially as well as axially. For example, the microporous section need not extend about the entire circumference of the extensible member 612. FIGURE 13 illustrates an alternative embodiment in which the microporous portion 640f generally extends about one-half of the circumference of the extensible member to form an arc-shaped section that is permeable to fluid within the extensible member 612. An impermeable arc-shaped section 650 is positioned adjacent the arc-shaped microporous portion 640f. Additionally, two or more microporous portions can be spaced circumferentially about the extensible member 612. FIGURE 14 shows an alternative embodiment including four equally spaced arcuate microporous portions 640g. Arc- shaped impermeable portions 652 are spaced between the microporous portions 640g. As in the case of the embodiments described above, the microporous portions 640g can be equally spaced and of similar size, as illustrated in FIGURE 14, or, in the alternative, can distinctly sized and spaced. Further, the number of microporous portions described is exemplary only; the number of microporous portions is limited only by the circumference of the extensible member 612 and the desired size of the microporous portions.

The impermeable sections of each of the embodiments described above can be constructed of the same microstructure as the microporous portions adjacent thereto, i.e. nodes having the same size and orientation. The impermeable sections can be made impermeable by sealing the sections with a coating provided on either the inner surface 634 or the outer surface 632, or both, to inhibit or prevent fluid flow through the sections. Alternatively, the microstructure of the impermeable sections can be constructed to have an inherently impermeable, non-porous structure. For example, the nodes forming the microstructure of the impermeable sections can be spaced apart or oriented to inhibit or prevent fluid from passing therethrough.

Various fluoropolymer materials are suitable for use in the fluid delivery device of the present invention. Suitable fluoropolymer materials include, for example, polytetrafluoroethylene (PTFE) or copolymers of tetrafluoroethylene with other monomers may be used. Such monomers include ethylene, chlorotrifluoroethylene, perfluoroalkoxytetrafluoroethylene, or fluorinated propylenes such as hexafluoropropylene, ePTFE is the preferred material of choice. Accordingly, while the fluid delivery device 600 can be manufactured from various fluoropolymer materials, and the manufacturing methods of the present invention can utilize various fluoropolymer materials, the description set forth herein refers specifically to ePTFE.

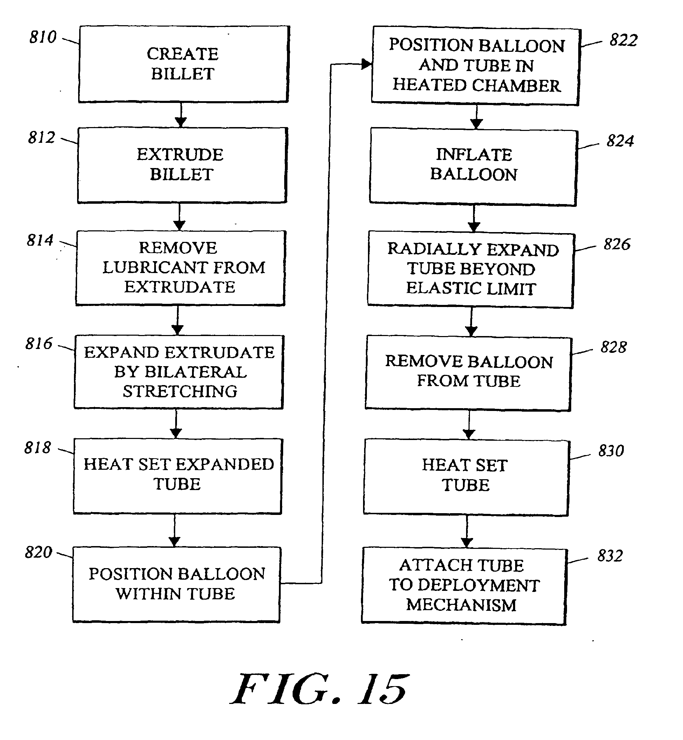

A method of manufacturing a fluid delivery device in accordance with the present invention will be described in connection with the flow chart shown in FIGURE 15. The fluid delivery device 600 is manufactured in a manner similar to that of the radially expandable device 10 described above. The fluid delivery device 300 of the present invention is produced from a tube constructed of expanded fluoropolymer material, which is preferably produced through an extrusion and a longitudinal expansion process. The preferred fluoropolymer material is expanded PTFE (ePTFE), which is a hydrophobic, biocompatible, inelastic material having a low coefficient of friction, although, as discussed above, other inelastic, biocompatible fluoropolymer materials may be used.

To produce the ePTFE tube, a billet comprising a PTFE resin mixed with an organic lubricant is utilized. Various organic lubricants are suitable such as naphtha, ISOPAR-G and ISOPAR-H available from Exxon Corporation. The blended resin is compressed at low pressure to yield a tubular billet of PTFE resin and lubricant, step 810 of FIGURE 15. The tubular billet is then extruded through an extruder, for example a ram extruder, to reduce the cross section of the billet and to yield a tubular extrudate, step 812. The organic lubricant can be removed from the extrudate by drying the extrudate in a heated oven, step 814.

Once the tubular extrudate is produced, the extrudate is expanded by longitudinal stretching, step 716. Preferably, the extrudate is bilaterally stretched. Bilateral stretching is accomplished by displacing both ends of the extrudate, sequentially or simultaneously, away from the center of the extrudate. Bilateral stretching provides a material that is homogeneously stretched over the majority of its length and yields a uniform porosity over the length of the material. After the extrudate has been stretched, it is heat set to lock in the microstructure of the material, step 818 of FIGURE 15, and to complete the process of the forming the tube 710 of ePTFE.

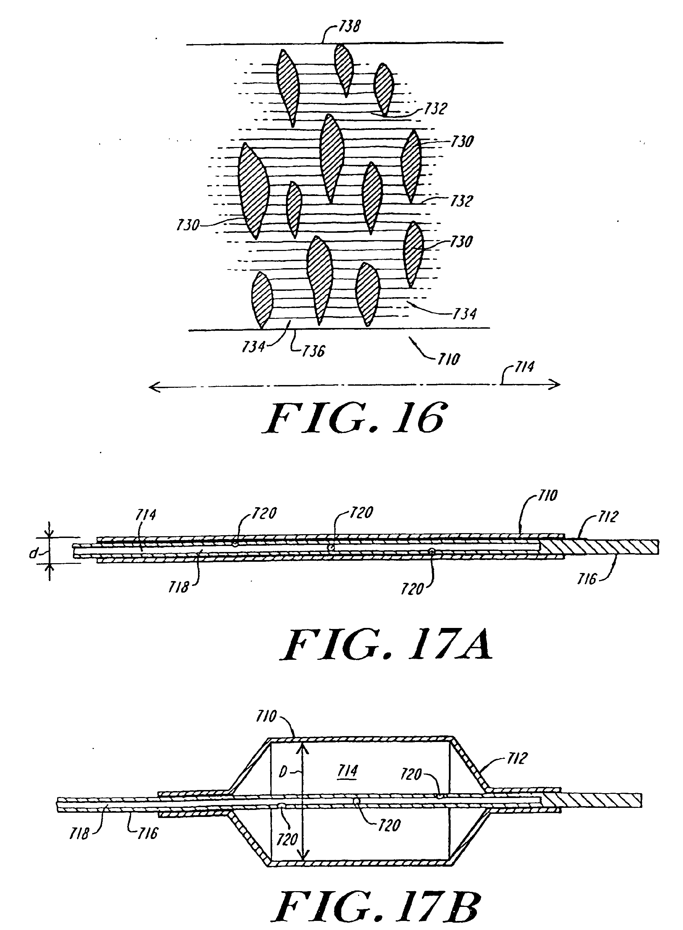

Referring to FIGURE 17B, is a schematic representation of the ePTFE tube 710 as formed by the extrusion and expansion process described above is illustrated. For purposes of description, the microstructure of the tube 710 has been exaggerated. Accordingly, while the dimensions of the microstructure are enlarged, the general character of the illustrated microstructure is representative of the microstructure prevailing within the tube 710.

The microstructure of the ePTFE tube 710 is characterized by nodes 730 interconnected by fibrils 730. The nodes 730 are generally aligned with one another and are generally oriented perpendicular to the longitudinal axis 714 of the tube 710. Substantially all of the nodes 730 extend along a transverse axis 734 from an inner surface 736 to an outer surface 738 of the tube 710. This microstructure of nodes 730 interconnected by fibrils 730 provides a microporous structure having microfibrillar spaces which define generally aligned through-pores or channels 734 extending entirely from the inner wall 736 and the outer wall 738 of the tube 710. The through-pores 734 are perpendicularly oriented (relative to the longitudinal axis 734), internodal spaces that traverse from the inner wall 736 to the outer wall 738. The size and geometry of the through-passages can be altered through the extrusion and expansion process, to yield a microstructure that is impermeable, semi-impermeable, or permeable.

Although it is preferable for the micro-channels of the ePTFE tube 710, and the resultant fluid delivery device 600, to be oriented generally perpendicular to the longitudinal axis of the tube ePTFE 710 or fluid delivery device 600, other orientations of the micro-channels can be utilized. For example, by twisting or rotating the ePTFE tube during the extrusion and/or stretching process, the micro-channels can be oriented at an angle to an axis perpendicular to the longitudinal axis of the tube.

In a preferred embodiment, the ePTFE tube 710, and the resultant fluid delivery device 600, has a fine nodal structure that is uniform throughout the cross section and length of the ePTFE tube. The preferred uniform fine nodal structure provides the fluid delivery device 600 with improved expansion characteristics as the expandable device dependably and predictably expands to the second diameter. The preferred fine nodal structure is characterized by nodes having a size and mass less than the nodes found in conventional ePTFE grafts, preferably in the range of 25$\mu$m to 30 $\mu$m. Additionally, the spacing between the nodes, referred to as the internodal distance, and the spacing between the fibers, referred to as the interfibril distance, is also preferably less than found in conventional ePTFE grafts, preferably in the range of 1 $\mu$m to 5 $\mu$m. Moreover, the internodal distance and the interfibril distance in the preferred embodiment is preferably uniform throughout the length and the cross section of the ePTFE tube. The preferred uniform nodal structure can be created by forming the billet with a uniform lubricant level throughout its cross section and length. Stretching the tubular extrudate at higher stretch rates, for example at rates greater than 1 in/s, yields the preferred fine nodal structure. Preferably, the extrudate is stretched at a rate of approximately 10 in/s or greater.

Continuing to describe the manufacturing method of the present invention and referring to FIGURES 15 and 17A, the ePTFE tube 710, having an initial diameter d, is pulled over a balloon 712 to position the balloon 712 within the lumen 714 of the tube 710, step 820 of FIGURE 15. The balloon 712 is preferably constructed of an inelastic material such as, for example, PET, nylon, or PTFE, such that the balloon 712, when inflated, attains a predetermined size and shape. The balloon 712 can be bonded or otherwise coupled to a rigid catheter or hypo-tube 716 to facilitate placement and removal of the ePTFE tube as described below. The catheter 716 has a central inflation lumen 718 and a plurality of side-holes 720 to provide for the delivery of an inflation fluid to inflate the balloon 712. Prior to placement of the ePTFE tube 710 over the balloon 712, a small amount of negative pressure (vacuum) can be applied to the balloon 712 to reduce the balloon to a minimum deflated profile.

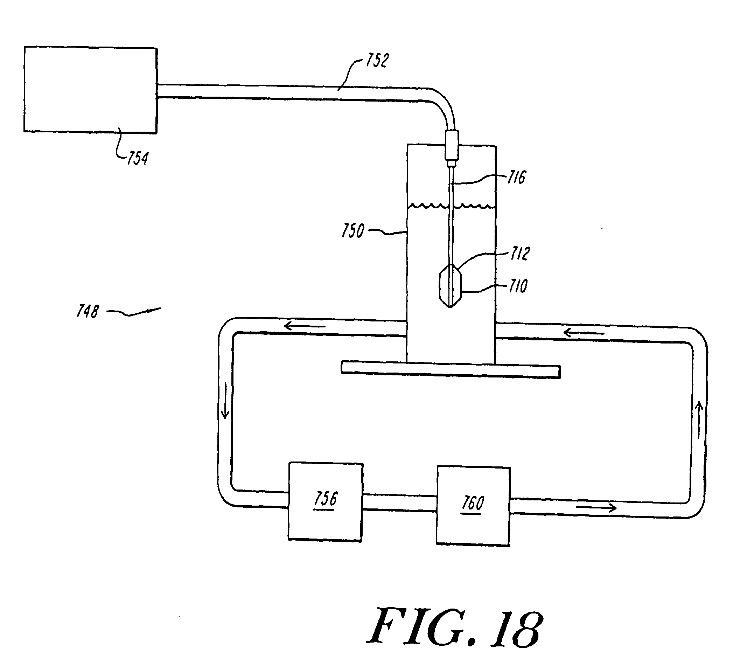

Referring to FIGURE 18, a system 748 for inflating the balloon 712 within the ePTFE tube is illustrated. The balloon 712 and the ePTFE tube 710 are positioned within water heated chamber 750, step 822 of FIGURE 15. The catheter 716 is connected by plastic tubing 752 to a pump 754, such as an ISCO syringe pump, for inflation of the balloon 712 with a fluid. Heated water from a circulating temperature bath 758 is pumped through the chamber 750 by a pump 760 to maintain the water within the chamber 750 at a desired temperature. The water within the chamber 750 is heated to a temperature between approximately 35°C and approximately 60°C. The preferred temperature of the water within the chamber 750 is 50°C. The constant, elevated temperature provided by the circulating water can contribute to uniform expansion, both circumferentially and longitudinally, of the ePTFE balloon, as well as uniform wall thickness.

Referring to FIGURES 17B and 18, the balloon 712 can be inflated by introduction of pressurized fluid from the pump 754 to the lumen 714 of the ePTFE tube 710. The overlying ePTFE tube 710 expands with the inelastic balloon722 until both the balloon 712 and the ePTFE tube 710 obtain the predetermined size and shape of the inflated balloon 712, step 824 of FIGURE 15. The inflated balloon 712 thus imparts its predetermined size and shape to the ePTFE tube 710. This radially expansion process is referred to as "blow-molding". The PTFE tube 710 shown in FIGURE 17B is radially expanded from the initial diameter d (FIGURE 17A) to an increased diameter D.

it is preferable for the ePTFE tube 710 to be plastically deformed by the radial expansion of the inelastic balloon 712, step 826 of FIGURE 15. The terms "plastic deformation" and "plastically deform," as used herein is intended to include the radial expansion of the ePTFE tube 710 beyond the elastic limit of the ePTFE material such that the ePTFE material is permanently deformed. Once plastically deformed, the ePTFE material forming the tube 710 becomes substantially inelastic, i.e., the ePTFE tube generally will not, on its own, return to its pre-expansion size and shape.

The ePTFE tube 710 can be removed from the balloon 712 by removing the inflation fluid from the balloon 712 using the pump754 and sliding the ePTFE tube 710 relative to balloon 712 and catheter 716, i.e. in the direction of arrows A in FIGURE 17C, step 828 of FIGURE 15. The tube 710 can be heat set at a temperature above the sinter point of the material forming the tube, 360°C for ePTFE, to lock in the structure of the tube 710, step 830 of FIGURE 15. The pump 750 can be used to provide a slight vacuum within the balloon 712 to facilitate removal of the ePTFE tube 710.

The ePTFE tube 710 can be attached to a deployment mechanism such as hollow tube 620 described above, step 832 of FIGURE 15. A suitable adhesive can be used to secure the ePTFE tube to the deployment mechanism.

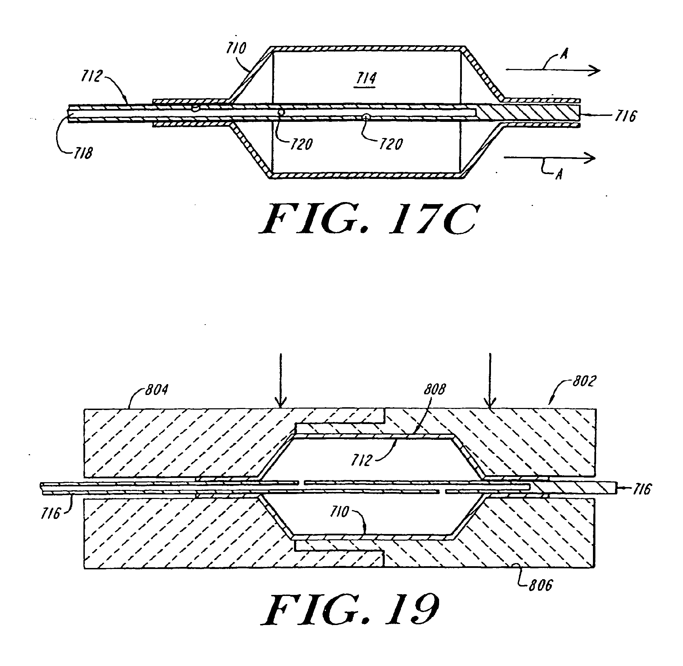

Referring to FIGURE 19, an alternative method of manufacturing a fluid delivery device employing a mold 802 is illustrated. The mold 802 includes two interconnected sections 804 and 806 forming an internal mold cavity 808 for receiving the ePTFE tube 710 with the balloon 712 positioned therein. The mold 802 is preferably constructed of a rigid, unyielding material such as a metal or metal alloy. Suitable metals or metal alloys include brass and steel alloys. The internal mold cavity 808 preferably has a size and shape analogous to that of the inflated balloon 712 to ensure that the inflated balloon 712, and the overlying ePTFE tube 710 concentrically expand.

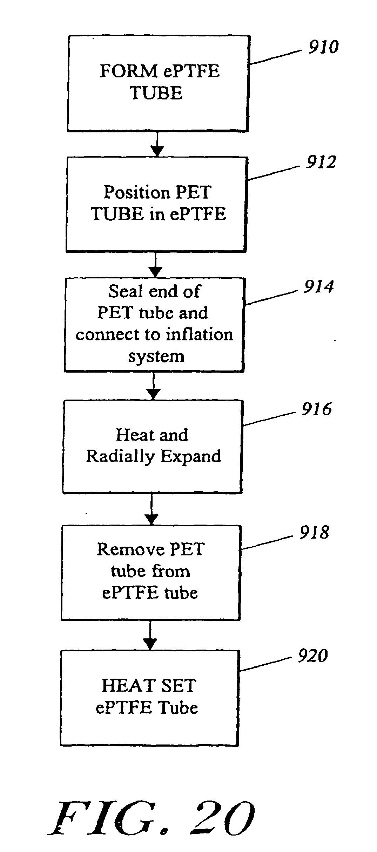

Referring to the flow chart illustrated in FIGURE 20, a further alternative method of manufacturing a fluid delivery device according to the teachings of the present invention will be described. A tube constructed of ePTFE is formed in accordance with the methods described above, step 910. A tube formed of an extruded inelastic material such as PET is used in place of balloon 712 to radially expand the ePTFE tube. The extruded tube is positioned within the ePTFE tube 710, step 912. The extruded tube is then sealed at one end and attached to an inflation system at the other end, step 914. The extruded tube can then be inflated by an inflation medium to radially expand the ePTFE tube, step 916. The extruded tube and ePTFE tube are preferably heated to the glass transition temperature of the extruded tube, approximately 80°C - 100°C for PET, as the extruded tube is inflated within the ePTFE tube. It is preferable to limit the temperature of the extruded tube to a temperature less than or equal to the glass transition temperature of the material forming the extruded tube to facilitate removal of the extruded tube from the ePTFE tube. Heating the extruded to a temperature above the glass transition temperature will cause the extruded tube to heat set in an expanded configuration, which makes removing the extruded tube from the ePTFE tube difficult.

After the extruded tube and ePTFE tube are expanded to desired size and shape, the extruded tube is deflated and removed from the ePTFE tube, step 918. The ePTFE tube is then heat set to lock in the structure of the ePTFE tube, step 920.

A mold, such as mold 802, can be employed during radial expansion of the ePTFE tube using the PET tube. The mold is preferably heated within the hot water chamber of an inflation system, such as inflation system 748 illustrated in FIGURE 18, or by other means such as a hot oil bath or through a steam, hot air, electric, radio frequency or infra red heat source. The mold can be constructed of a material having good head transfer characteristics, such as metal or metal alloy, for example brass. The mold includes a mold cavity having a size and shape analogous to the desired size and shape of the fluid delivery device 600 in the second diameter configuration.

The resultant radially expanded ePTFE tube 710, produced in accordance with the above described methods, provides a radially expandable fluid delivery device, such as the fluid delivery device 600 illustrated in FIGURES 9 and 10 and described above, that is radially expandable from a relaxed, collapsed diameter to the second, increased diameter D upon application of a radial deployment force from a deployment mechanism within the tube 710. Moreover, the microstructure of the radially expanded ePTFE tube 710 can be manufactured through the process of the present invention to have a porosity sufficient to permit fluid to flow therethrough. In particular, the microstructure of the resultant fluid delivery device is analogous to the microstructure of the ePTFE tube 710, i.e. nodes interconnected by fibrils, the space between the nodes defining micro-channels through the wall of the device.

One feature of the manufacturing processes of the present invention is that the microporous structure of the ePTFE tube 710 forming the fluid delivery device 600 can be manipulated by varying the extrusion and expansion process parameters to produce different porosity characteristics. For example, the longitudinal stretch ratio of the ePTFE tube 710, i.e., the ratio of final stretched length of the tube to the initial length, and the diametric stretch ratio of the ePTFE tube 710, i.e., the ratio of the final diameter, after longitudinal stretching, and the initial diameter, and the stretch rate can be varied to yield fluid recovery devices having different porosity. Applicants determined that larger longitudinal stretch ratios, in the order of 2:1 to 3:1, can result in a ePTFE tube having a microstructure characterized by increased internodal distances and interstitial space, i.e., the micro-channels within the microstructure of the ePTFE tube are increase in sized. Suitable longitudinal stretch ratios can be from 1.1:1 to 10:1.

Fluid delivery devices formed from ePTFE tubes having larger stretch ratios require minimal fluid pressure within the device to achieve the desired flow rate of fluid through the walls of the device. Such fluid delivery devices will consequently provide little radially-outward dilation force, which can be advantageous for some applications such as delivering therapeutic or diagnostic agents to healthy tissue. Conversely, ePTFE tubes having smaller stretch ratios result in fluid delivery devices that are less porous, as the internodal distances are reduced, and, thus, generally require increased fluid pressure to deliver the fluid. Such fluid delivery devices consequently provide increased radially outward dilation force, which can be advantageous for treatment applications such as reducing arterial lesions with pressure. Accordingly, the porosity of the ePTFE tube, and hence the porosity of the resultant fluid delivery device, is dependent on the stretch ratios of the expansion process. By manipulating the stretch ratios of the ePTFE tube, i.e. increasing or decreasing the stretch ratios, the porosity of the fluid delivery device can be tailored to the specific treatment application.

The porosity of the ePTFE tube, and the subsequent fluid delivery device, can also depend on the density, the viscosity, the molecular weight, and the amount of lubricant used to form the billet used in the extrusion and expansion process. Increasing the amount by weight of lubricant or increasing the density or molecular weight of the lubricant, results in an extrudate having increased internodal distances and interstitial spaces prior to the step of expansion. Once stretched, the resultant ePTFE tube will reflect the increased porosity.

The process described above can be used to produce fluid delivery devices having a uniform porosity though out the length of the device. To produce discrete microporous portions within the fluid delivery devices a number of different processes can be employed. For example, a coating can be applied at select locations to the inner and/or outer surfaces of the fluid delivery device to produce impermeable sections within the device. The coating preferable seals the microchannels within the microstructure of the device to inhibit or prevent fluid from passing therethrough.

Alternatively, the porosity of the ePTFE tube can be selectively reduced subsequent to the step of expansion by applying heat to the select sections of the tube to return the sections to the pre-stretched porosity. The selectively heated sections will thus yield sections of reduced porosity. By controlling the amount of heat applied, the sections of reduced porosity can be made semi-permeable or impermeable to fluid.

In addition to selecting and varying the porosity of the fluid recovery device through the manufacturing process of the present invention, the parameters of the manufacturing process can be varied to produce an ePTFE tube having distinct expansion characteristics. The size and shape of the fluid recovery device of the present invention can also be varied.

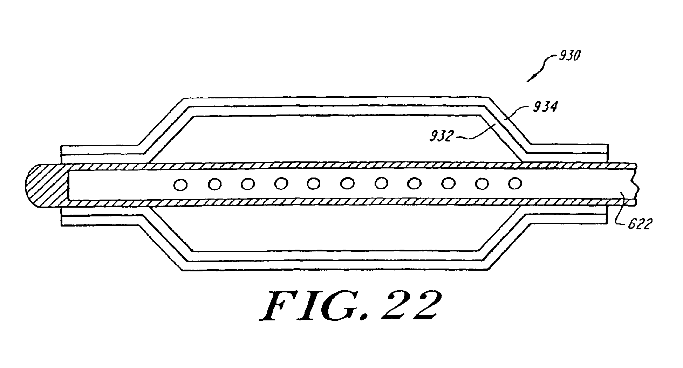

FIGURE 21 illustrates an exemplary embodiment of the fluid delivery device of the present invention in which the fluid delivery device 600 of FIGURE 9 is utilized as a catheter deployed dilation and diffusion balloon 850 for the treatment of a blood vessel 852 partially occluded by plaque deposits 856 adhered to the walls 858 of the blood vessel. This procedure is generally referred to as a Percutaneous Transluminal Angioplasty (PTA) procedure. The balloon 850 can be manufactured in accordance with the methods of the present invention and is shown in the expanded configuration. The ends 860 of the dilation balloon 850 are bonded to a catheter tube 862, which is used to provide an inflation fluid to the balloon 850 to effect expansion of the balloon 850 to a predefined and fixed maximum diameter. A therapeutic agent, such as antisense oligonucleotides (AS-ODNs), can be delivered through the walls of the balloon with the inflation fluid. The AS-ODNs are delivered locally to the walls of the body vessel to suppress restenosis of the body vessel subsequent to the PTA procedure.

A variety of therapeutic agents can be delivered with the fluid delivery device of the present invention. Exemplary therapeutic agents include: thrombolytics, antibiotics, chemotherapeutics, surfactants, diagnostic agents, steroids, hot saline, vasodilators, vasoconstrictors, and embolic agents. The localized delivery of thrombolytics, such as heparin, and urokinase, directly to the surface of thrombosed grafts or native body vessels can inhibit the-clotting of the graphs or native body vessels. The fluid delivery device of the present invention thus can be used as thrombolectomy balloon catheter to remove obstructions from grafts or native body vessels while concomitantly delivering thrombolytics to the walls of the graft or native body vessel.

The fluid delivery device of the present invention can further be used for the localized delivery of high concentrations of antibiotics to treatment sites such as the ear canal, the throat, and the upper respiratory passages. Anti-cancer agents such as chemotherapeutics delivered using the fluid delivery device of the present invention can increase the effectiveness and negate the systemic side effects of the anti-cancer agents. Exemplary forms of cancer targeted for this application can include: rectal, esophageal, and lung cancer. Surfactants can be delivered directly to the lungs using the fluid delivery device of the present invention to treat cystic fibrosis or hyaline membrane disease. Diagnostic agents can be delivered during angiography procedures through the fluid delivery device of the present invention to improve the visibility of contrast agents delivered locally to the treatment site. The local delivery of steroids such as anabolic steroids, using the fluid delivery device of the present invention can increase the effectiveness, e.g., promote muscle recovery, and negate systemic side effects such as lung and adrenal gland vantage.

The fluid delivery device of the present invention can also be provided with hydrophilic or hydrophobic coatings dependant on the particular treatment applications. For example, fluid delivery devices constructed of ePTFE, a naturally hydrophobic material, can be coated with a hydrophilic coating to provide the fluid delivery device with a hydrophilic outer surface. A suitable hydrophilic coating is PHOTOLINK available from Surmodics of Eden Prairie, MN.

In an alternative embodiment illustrated in FIGURE 22, the fluid delivery device of the present invention can be provided with multiple layers. The multi-layer fluid delivery device 930 includes a first layer 932 of biocompatible material, such as ePTFE, and a second layer 934 of biocompatible material that overlies the first layer 932. The second layer 934 can be constructed from the same or a different biocmopatible material than the first layer 932. As shown in FIGURE 22, the first layer 932 and the second layer 934 are preferably coexentsive, however, the second layer 934 need not extend the entire length of the first layer 932. Likewise, the first layer 932 may be smaller in length than the second layer 934.

The first layer 932 and the second layer 934 can each be formed from a separate ePTFE tube. The ePTFE tubes can be constructed in accordance with the manufacturing methods of the present invention such that each tube is characterized by a microstructure of nodes interconnected by fibrils. Preferably the nodes are oriented generally perpendicular to the longitudinal axis. The two ePTFE tubes can then be coaxially disposed and heated to bond the two tubes together. The bonded tubes can be radially expanded by a balloon in accordance with the above manufacturing methods of the present invention. By producing the ePTFE tubes forming the first and second layers using different process parameters, e.g. different stretch ratios or stretch rates, the distance between the nodes can varied between the ePTFE tubes. For example, the internodal distances of the microstructure of the first layer 932 can be generally greater than the internodal distances of the microstructure of the second layer 934. In this manner, the porosity of the first layer 932 and the porosity of the second layer 934 can be different.

Although the multi-layer fluid delivery device 930 illustrated in FIGURE 22 includes two layers, additional layers can be provided. Additional layers can be formed from further ePTFE tubes or through other methods, such as by wrapping ePTFE film about the first layer and/or second layer.

Example 1

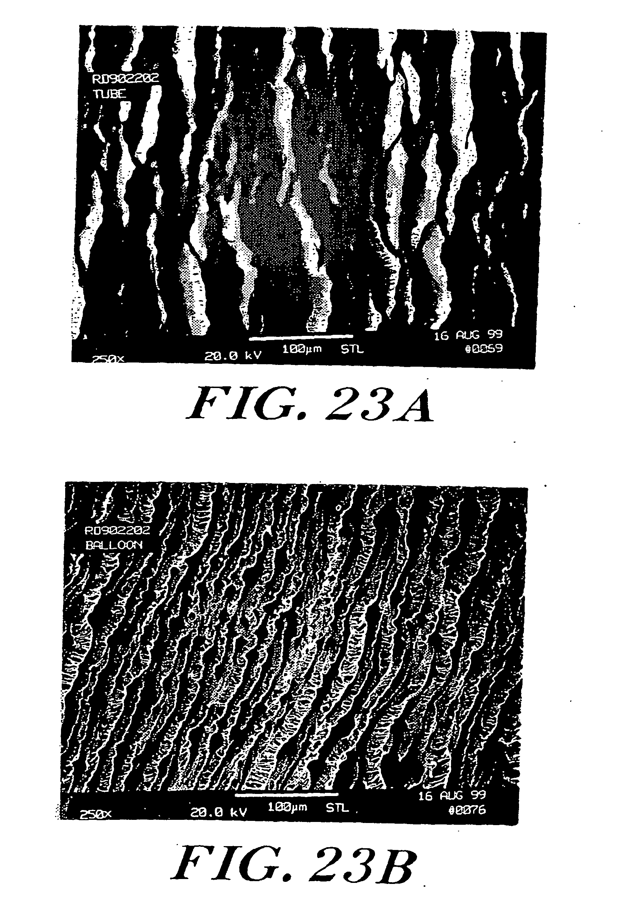

An exemplary fluid delivery device was constructed according to the manufacturing processes of the present invention by employing .068" (ID)/088" (OD) ePTFE tubing. The longitudinal stretch ratio of the ePTFE tube was 1.5:1, at a stretch rate of 10 inches per second. An electron micrograph of an exterior section of a sample ePTFE tube, after stretching, is shown in FIGURE 23A. FIGURE 23A illustrates the microstructure of nodes interconnected by fibrils of the ePTFE tubing and, in particular, the orientation of the nodes generally perpendicular to the longitudinal axis of the tubing and the internodal through-pores. An 8 mm x 8 cm PET balloon was employed to radially expand the ePTFE tubing. The PET balloon was attached to a vacuum source and a slight vacuum was placed on the PET balloon, about -5 to -10 psi. The ePTFE tubing was then positioned over the deflated PET balloon. The PET balloon and the ePTFE tubing were then connected to a hypo-tube and placed into a water heated chamber. Saline was injected into the PET balloon at a constant flow rate of about 10-15 ml/min, When the pressure within the balloon reached 70-80% of the rated balloon pressure, about 12-15 atm for the PET balloon employed, the flow rate was decreased to 2 ml/min. The balloon was then brought to its rated balloon pressure. The water was then removed from the PET balloon using the pump until a slight vacuum existed within the PET balloon (-5 to -10 psi). The hypo-tube was then removed from the chamber and the ePTFE tube was withdrawn from the balloon. The proximal I.D. of the resultant ePTFE tube was approximately 2/3 Fr larger than the distal I.D.

The resultant ePTFE balloon was then allowed to completely dry. The ePTFE balloon was cut to a desired length and the proximal tail of the ePTFE balloon was dipped into Loctite 7701 Prism Primer, available from Loctite, Corp. of Rocky Hill, CN. The distal tail of the ePTFE balloon was then dipped into the primer. The primer was allowed to evaporate for at least two minutes. The ePTFE balloon was then placed on a 5 Fr- 4 Fr tipped catheter by pulling the proximal end of the ePTFE balloon onto the 5 Fr end of the catheter body until the distal (4 Fr) end is about 0.1" from the tip of the catheter. The location of the proximal tail of the ePTFE balloon was then marked on the catheter and the ePTFE balloon was removed from the catheter. Loctite 4011 adhesive was then applied to the catheter shaft at a location slightly distal to the marked position. The ePTFE balloon was then slid back onto the catheter shaft until the proximal end was aligned with the mark. The adhesive was allowed to dry for one minute. A small volume of Loctite 4011 adhesive was dispensed onto the distal end of the catheter shaft adjacent to the distal end of the ePTFE balloon. This adhesive was drawn into the gap between the catheter and the balloon by capillary forces. The adhesive was then allowed to dry for at 24 hours.

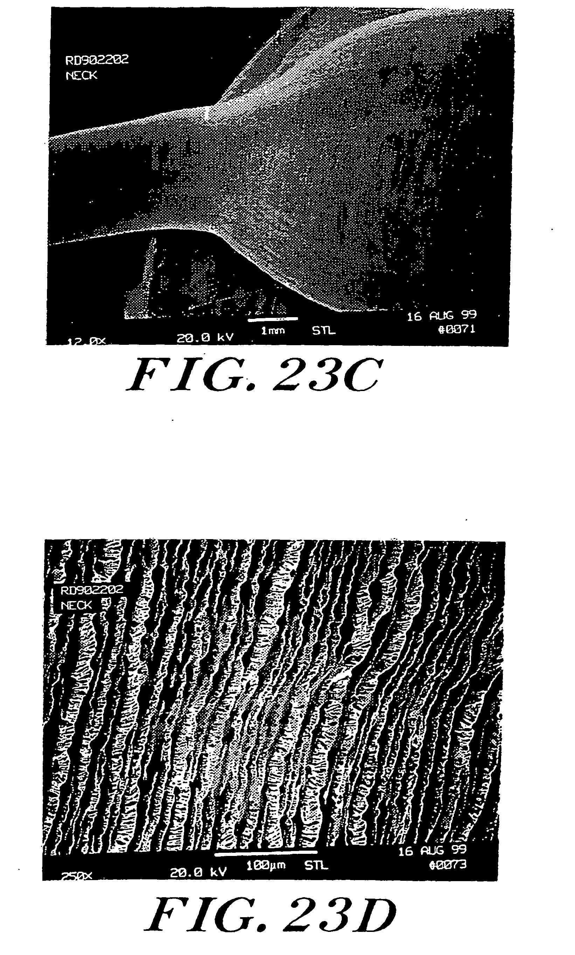

An electron micrograph of an exterior section of a sample ePTFE balloon is shown in FIGURES 23B-23D. FIGURE 23B illustrates the microstructure of nodes interconnected by fibrils of the body of the ePTFE balloon. FIGURES 23C and 23D are cross-sections of the neck of the ePTFE balloon at varying magnifications. FIGURES 23B-24D illustrate the internodal through-pores or channels of the ePTFE balloon as defined by the microfibrillar spaces between the nodes. The through-pores are oriented generally perpendicular to the longitudinal axis of the ePTFE balloon and provide fluid pathways between the inner surface and the outer surface of the ePTFE balloon.

Example 2

Three ePTFE synthetic grafts were treated using an ePTFE diffusion balloon constructed in accordance with the method of Example 1 to determine the permeability of a contrast agent into the grafts. The diffusion balloon employed was 8 cm in length, had an OD of 6 mm and was constructed from an 0.088" (OD)/.068" (ID) ePTFE tube having a stretch ratio of 1.5:1. Each of the grafts treated was an Atrium Hybrid PTFE graft, model no. 01200670, available from Atrium Medical of Hudson, NH. The grafts had a 6mm inner diameter and a wall thickness of .025 inches. In each case, the balloon was inserted into a graft and inflated by infusing a contrast agent. The balloon was inflated to 1 atm. by the infused fluid. The balloon was then removed from the graft and the graft was flushed with water delivered at 500 ml/min. The contrast agent used was Hypaque-76 available from Mycomed, Inc. Table 1 summarizes each procedure.

Table 1

| Graft Sample | Duration of Balloon Infusion | Balloon Infusion Fluid | Graft Flushing Duration |
|---|---|---|---|
| 1 | 15 seconds | Saline & Contrast Agent | 5 minutes |
| 2 | 15 seconds | Saline & Contrast Agent | 30 seconds |
| 3 (Control) | 15 seconds | Saline | No Flushing |

X-rays were taken of each of the grafts samples to determine if the contrast agent was present in the grafts post infusion and perfusion (flushing). Table 2 summarizes the results of the X-rays for each graft.

Table 2

| Graft Sample | Presence of Contrast Agent |
|---|---|
| 1 | Yes |
| 2 | Yes |
| 3 | No |

Contrast agent was found in the walls of the both ePTFE graft samples infused with contrast agent by the diffusion balloon of the present invention, but not in the control sample. In graft samples 1 and 2, the contrast agent was found to have permeated through the entire wall of the graft. Flushing the grafts with water post infusion was determined to have no effect on the presence and concentration of the delivered contrast agent.

Example 3

The permeability of several ePTFE fluid delivery devices constructed in accordance with the manufacturing processes of the present invention was evaluated and compared with the permeability of selected synthetic grafts. Each of the fluid delivery devices was constructed from an extruded and expanded ePTFE tube as set forth in Table 3.

Table 3

| Fluid Delivery Device Sample | Stretch Ratio of ePTFE tube | Stretch Rate of ePTFE tube (in/s) | Wall Thickness of Fluid Delivery Device (in) | Surface Area of Fluid Delivery Device (in$^2$) |
|---|---|---|---|---|
| 1 | 1.5:1 | 20 | 0.0068 | 0.456 |
| 2 | 1.75:1 | 10 | 0.0055 | 0.469 |
| 3 | 3:1 | 10 | 0.005 | 0.469 |
| 4 | 5.1 | 10 | 0.003 | 0.503 |

Table continued

| Fluid Delivery Device Sample | Stretch Ratio of ePTFE tube | Stretch Rate of ePTFE tube (in/s) | Wall Thickness of Fluid Delivery Device (in) | Surface Area of Fluid Delivery Device (in$^2$) |
|---|---|---|---|---|
| 5 | 1.25:1 | 10 | 0.0075 | 0.399 |
| 6 | 1.5:1 | 0.5 | 0.0055 | 0.399 |
| 7 | 1.5:1 | 10 | 0.005 | 0.399 |
| 8 | 1.5:1 | 10 | 0.005 | 0.29 |

The synthetic grafts analyzed were standard and thin wall grafts available from Atrium Medical Corporation and W. L. Gore & Associates, Inc. Information regarding the grafts is set forth in Table 4

Table 4

| Graft sample | Type | Wall Thickness (in) | Surface Area (in$^2$) |
|---|---|---|---|
| A | Atrium 6mm std | 0.025 | 0.964 |
| B | Atrium 6mm thin | 0.022 | 0.963 |
| C | Gore 7mm thin | 0.016 | 1.143 |
| D | Gore 6 mm std | 0.024 | 0.963 |
| E | Gore 4mm | 0.025 | 0.59 |
| F | Atrium 4mm | 0.02 | 0.49 |

The fluid delivery devices and the synthetic grafts were infused with a fluid to determine the permeability of the samples. The permeability of each of the samples is reflected by the hydrodynamic resistance and the hydraulic conductivity data set forth in Table 5.

Table 5

| Sample | Hydrodynamic resistance (psi*min*mL$^{-1}$) | Hydraulic Conductivity (cm$^4$/ (dyne* s)* 10$^{12}$) |
|---|---|---|
| 1 | 21.2 | 67 |
| 2 | 7.18 | 155 |
| 3 | 2.76 | 368 |
| 4 | 2.81 | 202 |
| 5 | 13.4 | 134 |
| 6 | 1.59 | 825 |
| 7 | 1.5 | 795 |
| 8 | 1.7 | 966 |
| A | 2.394 | 1031 |
| B | 1.03 | 2112 |
| C | 0.711 | 1874 |
| D | 0.623 | 3808 |
| E | 1.5 | 2673 |
| F | 1.2 | 3207 |

Hydraulic conductivity was determined using Darcy's Law for describing stead, flow through a porous media. Darcy's law is defined in Equation I as

$$Q/A = -K(\Delta P/\Delta X), \qquad (Eq. 1)$$

where Q is flow rate, A is cross sectional area for flow. i.e. the surface area of the sample, P is pressure, X is wall thickness, and K is the hydraulic conductivity.

Applicants determined that the permeability of a fluid delivery device relative to the permeability of the vessel being treated is an important consideration in establishing effective fluid delivery into the walls of the vessel. In particular, for a given fluid flow rate and pressure, a greater volume of fluid will penetrate the walls of a vessel being treated if the permeability of the vessel is greater than the permeability of the fluid delivery device. Accordingly, it is desirable for the permeability of the fluid delivery device to be less than the permeability of the vessel being treated. The permeability of the fluid delivery device can be tailored to be less than the vessel being treated by varying the process parameters of the manufacturing processes described above. The process parameters include lubricant density, lubricant viscosity, lubricant molecular weight, longitudinal stretch ratio, and stretch rate.

The permeability data set forth in Table 5 illustrates that the permeability, when measured in terms of the hydraulic conductivity, for each of the fluid delivery devices tested was less than the permeability of each of the synthetic grafts tested. This indicates that the Applicants' fluid delivery device may be particularly effective for delivering fluid to the walls of synthetic grafts.

Applicants have determined that it is desirable for the hydraulic conductivity of the fluid delivery device to be less than $1000$ ($cm^4/ (dyne* s)* 10^{12}$) for treatment of synthetic grafts. Preferably, the fluid delivery device has a hydraulic conductivity of less than $500$ ($cm^4/ (dyne* s)* 10^{12}$) . For treatment of natural body vessels and grafts, it is preferable for the hydraulic conductivity of the fluid delivery device to be less than $100$ ($cm^4/ (dyne* s)* 10^{12}$).

It will thus be seen that the invention efficiently attains the objects made apparent from the preceding description. Since certain changes may be made in the above constructions without departing from the scope of the invention, it is intended that all matter contained in the above description or shown in the accompanying drawings be interpreted as illustrative and not in a limiting sense.

It is also to be understood that the following claims are to cover all generic and specific features of the invention described herein, and all statements of the scope of the invention which, as a matter of language, might be said to fall therebetween.

## Claims

1. A radially expandable catheter balloon device comprising:

    a balloon constructed of a fluoropolymer material, the balloon having a longitudinal axis and a wall having a thickness transverse to the longitudinal axis, the wall having a microstructure of nodes interconnected by fibrils, substantially all the nodes being oriented generally perpendicularly to the longitudinal axis of the balloon along at least a portion of the balloon, the balloon being deployable from a reduced diameter, collapsed configuration to an increased diameter, expanded configuration upon application of an expansion force.

2. The device of claim 1, wherein the balloon is expandable to a pre-defined and fixed increased diameter that is generally independent of the expansion force used to expand the device to the expanded configuration.

3. The device of claim 1, wherein the fluoropolymer material is expanded polytetrafluoroethylene (ePTFE).

4. The device of claim 1, wherein the balloon is tubular in shape and wherein the wall extends radially between an inner and an outer surface.

5. The device of claim 4, wherein the nodes are oriented such that spaces between the nodes form channels oriented and extending from the inner surface to the outer surface of the wall.

6. The device of claim 1, wherein the nodes are separated by an internodal distance, the internodal distance being approximately $1\mu m$ - $150\mu m$.

7. The device of claim 1, wherein the balloon is monolithic in construction.

8. The device of claim 1, wherein the catheter balloon device is a medical treatment device.

9. The device of claim 8, wherein the medical treatment device is suitable for deployment within a body vessel by a catheter.

10. The device of claim 8, wherein the balloon of the medical treatment device is sized and shaped to facilitate removal of obstructions from the body vessel.

11. A medical treatment catheter balloon device comprising:

a catheter having an elongated hollow tube defining an inflation lumen extending from a proximal end to a distal end, and a balloon constructed of a biocompatible fluoropolymer material and attached to the distal end of the tube, the balloon having a wall having a thickness extending between an inner and an outer surface and a lumen in fluid communication with the inflation lumen of the catheter, the wall having a microstructure of nodes interconnected by fibrils, the balloon being deployable from a first, reduced diameter configuration to a second, increased diameter configuration, upon application of an expansion force wherein the wall of the balloon includes at least one microporous portion having a porosity sufficient for a fluid to permeate through the wall, substantially all of the nodes within the microporous portion being oriented substantially perpendicular to the longitudinal axis of the balloon.

12. A radially expandable fluid delivery catheter balloon device having a longitudinal axis and a wall transverse to the longitudinal axis, the fluid delivery device comprising: a first layer of biocompatible material having a microstructure of nodes interconnected by fibrils, and a second layer of biocompatible material having a microstructure of nodes interconnected by fibrils, the second layer overlying the first layer, the wall of the fluid delivery device extending between an inner surface of the first layer and an outer surface of the second layer, the fluid delivery device being deployable from a first, reduced diameter configuration to a second, increased diameter configuration, upon introduction of pressurized fluid wherein the wall of the fluid delivery device includes at least one microporous portion having a porosity sufficient for a fluid to permeate through the wall, substantially all of the nodes within the microporous portion being oriented such that spaces between the nodes form generally aligned micro-channels oriented and extending from the inner surface of the first layer to the outer surface of the second layer, the micro-channels being sized to permit fluid to permeate from the inner surface of the first layer to the outer surface of the second layer.

13. The fluid delivery device of claim 12, wherein substantially all of the nodes within the microporous portion being are substantially perpendicular to the longitudinal axis of the member.

14. The fluid delivery device of claim 12, wherein the mico-channels within the first layer are sized differently than the mico-channels within the second layer.

15. The fluid delivery device of claim 12, wherein the nodes in the first layer are separated by a first internodal distance and the nodes in the second layer are separated by a second internodal distance, wherein the first internodal distance is different from the second internodal distance.

16. The fluid delivery device of claim 12, wherein the biocompatible material of the first layer is different than the biocompatible material of the second layer.

**Patentansprüche**

1. Radial aufweitbare Ballonkathetervorrichtung, umfassend
einen aus einem Fluorpolymermaterial gefertigten Ballon, wobei der Ballon eine Längsachse und eine Wand mit einer Dicke quer zur Längsachse aufweist, wobei die Wand eine Mikrostruktur von untereinander mittels Fibrillen verbundenen Knoten aufweist, wobei die Knoten im wesentlichen alle allgemein senkrecht zur Längsachse des Ballons entlang mindestens eines Abschnitts des Ballons ausgerichtet sind, wobei der Ballon nach Aufbringen einer Ausdehnungskraft von einem zusammengefalteten Zustand mit verringertem Durchmesser in einen aufgeweiteten Zustand mit vergrößertem Durchmesser entfaltbar ist.

2. Vorrichtung nach Anspruch 1, wobei der Ballon auf einen vordefinierten und festgelegten vergrößerten Durchmesser aufweitbar ist, der im allgemeinen unabhängig von der Ausdehnungskraft ist, die aufgebracht wird, um die Vorrichtung in den aufgeweiteten Zustand zu expandieren.

3. Vorrichtung nach Anspruch 1, wobei das Fluorpolymermaterial expandiertes Polytetrafluorethylen (ePTFE) ist.

4. Vorrichtung nach Anspruch 1, wobei der Ballon eine rohrförmige Gestalt aufweist und die Wand radial zwischen einer Innen- und einer Außenfläche verläuft.

5. Vorrichtung nach Anspruch 4, wobei die Knoten so ausgerichtet sind, dass Räume zwischen den Knoten Kanäle bilden, die von der Innenfläche zur Außenfläche der Wand ausgerichtet sind und verlaufen.

6. Vorrichtung nach Anspruch 1, wobei die Knoten durch einen internodalen Abstand voneinander getrennt sind, wobei der internodale Abstand etwa 1 $\mu$m bis 150 $\mu$m beträgt.

7. Vorrichtung nach Anspruch 1, wobei der Ballon monolithisch ausgebildet ist.

8. Vorrichtung nach Anspruch 1, wobei die Ballonkathetervorrichtung eine medizinische Behandlungsvorrichtung ist.

9. Vorrichtung nach Anspruch 8, wobei die medizinische Behandlungsvorrichtung zum Entfalten in einem Körpergefäß durch einen Katheter geeignet ist.

10. Vorrichtung nach Anspruch 8, wobei der Ballon der medizinischen Behandlungsvorrichtung so bemessen und geformt ist, dass er das Entfernen von Obstruktionen aus dem Körpergefäß erleichtert.

11. Medizinische Behandlungsballonkathetervorrichtung, umfassend
einen Katheter, aufweisend ein gestrecktes, hohles Rohr, das ein Aufblaslumen definiert, das sich von einem proximalen zu einem distalen Ende erstreckt, und einen Ballon, ausgebildet aus biokompatiblem Fluorpolymermaterial und sich anschließend an das distale Ende des Rohrs, wobei der Ballon eine Wand aufweist, die eine Dicke hat, die sich zwischen einer inneren und einer äußeren Oberfläche erstreckt, und ein Lumen, das in Flüssigkeitskommunikation mit dem Aufblaslumen des Katheters steht, wobei die Wand eine Mikrostruktur von untereinander mittels Fibrillen verbundenen Knoten aufweist, wobei der Ballon von einem ersten Zustand mit verringertem Durchmesser in einen zweiten Zustand mit vergrößertem Durchmesser nach Aufbringen einer Ausdehnungskraft entfaltbar ist, wobei die Wand des. Ballons mindestens einen mikroporösen Abschnitt einschließt, der eine Porosität aufweist, die ausreicht, um eine Flüssigkeit durch die Wand durchdringen zu lassen, wobei im wesentlichen alle Knoten innerhalb des mikroporösen Abschnitts im wesentlichen senkrecht zu der Längsachse des Ballons ausgerichtet sind.

12. Radial aufweitbare Flüssigkeitsabgabeballonkathetervorrichtung, aufweisend eine Längsachse und eine Wand quer zu der Längsachse, wobei die Flüssigkeitsabgabevorrichtung umfasst
eine erste Schicht aus biokompatiblem Material, aufweisend eine Mikrostruktur von untereinander mittels Fibrillen verbundenen Knoten, und eine zweite Schicht aus biokompatiblem Material, aufweisend eine Mikrostruktur von untereinander mittels Fibrillen verbundenen Knoten, wobei die zweite Schicht auf der ersten Schicht aufliegt, wobei sich die Wand der Flüssigkeitsabgabevorrichtung zwischen der inneren Oberfläche der ersten Schicht und der äußeren Oberfläche der zweiten Schicht erstreckt, wobei die Flüssigkeitsabgabevorrichtung von einem ersten Zustand mit verringertem Durchmesser in einen zweiten Zustand mit vergrößertem Durchmesser nach Einführen von unter Druck stehender Flüssigkeit entfaltbar ist, wobei die Wand der Flüssigkeitsabgabevorrichtung mindestens einen mikroporösen Abschnitt einschließt, der eine Porosität aufweist, die ausreicht, um eine Flüssigkeit durch die Wand durchdringen zu lassen, wobei im wesentlichen alle Knoten innerhalb des mikroporösen Abschnitts so ausgerichtet sind, dass die Räume zwischen den Knoten hauptsächlich in Linie gebrachte Mikrokanäle ausbilden, die von der inneren Oberfläche der ersten Schicht zu der äußeren Oberfläche der zweiten Schicht ausgerichtet sind und sich erstrecken, wobei die Mikrokanäle so bemessen sind, dass sie Flüssigkeit von der inneren Oberfläche der ersten Schicht zu der äußeren Oberfläche der zweiten Schicht durchdringen lassen.

13. Flüssigkeitsabgabevorrichtung nach Anspruch 12, wobei im wesentlichen alle Knoten innerhalb des mikroporösen Abschnitts im wesentlichen senkrecht zu der Längsachse des Bauteils sind.

14. Flüssigkeitsabgabevorrichtung nach Anspruch 12, wobei die Mikrokanäle innerhalb der ersten Schicht anders bemessen sind als die Mikrokanäle innerhalb der zweiten Schicht.

15. Flüssigkeitsabgabevorrichtung nach Anspruch 12, wobei die Knoten in der ersten Schicht durch einen ersten intemodialen Abstand getrennt sind und die Knoten der zweiten Schicht durch einen zweiten internodalen Abstand getrennt sind, wobei sich der erste internodale Abstand von dem zweiten internodalen Abstand unterscheidet.

16. Flüssigkeitsabgabevorrichtung nach Anspruch 12, wobei sich das biokompatible Material der ersten Schicht von dem biokompatiblen Material der zweiten Schicht unterscheidet.

**Revendications**

1. Dispositif à cathéter de ballon expansible radial comprenant:

   un ballon construit d'un matériel fluoropolymérique, ledit ballon ayant un axe longitudinale et un mur ayant un épaisseur perpendiculaire à l'axe longitudinale, ledit mur ayant une microstructure des noeuds interconnectés par des fibrilles où pour l'essentiel tous les noeuds sont orientés en général perpendiculairement à l'axe longitudinale du ballon au moins au long d'une partie de ballon, ledit ballon est déployable d'un diamètre réduit, un état collapsé à un diamètre agrandi, un état élargi par l'application d'une force d'expansion.

2. Dispositif selon la revendication 1, **caractérisé en ce que** le ballon est expansible à un diamètre agrandi prédéfini et fixé qui est en général indépendant de la force d'expansion utilisée pour élargir le dispositif à l'état élargi.

3. Dispositif selon la revendication 1, **caractérisé en ce que** le matériel fluoropolymérique est polytétrafluoroéthylène élargi (ePTFE).

4. Dispositif selon la revendication 1, **caractérisé en ce que** le ballon a une forme tubulaire et **caractérisé en ce que** le mur s'étend radialement entre une surface intérieure et extérieure.

5. Dispositif selon la revendication 4, **caractérisé en ce que** les noeuds sont orientés de cette façon que des espaces entre les noeuds forment des canaux orientés et s'étendant de la surface intérieure vers la surface extérieure du mur.

6. Dispositif selon la revendication 1, **caractérisé en ce que** les noeuds sont séparés par une distance internodale où la distance internodale a environ 1 $\mu$m - 150 $\mu$m.

7. Dispositif selon la revendication 1, **caractérisé en ce que** le ballon est construit monolithiquement.

8. Dispositif selon la revendication 1, **caractérisé en ce que** le dispositif à cathéter de ballon est un dispositif à traitement medical.

9. Dispositif selon la revendication 8, **caractérisé en ce que** le dispositif à traitement medical est approprié pour déployage dans un vaisseau du corps par un cathéter.

10. Dispositif selon la revendication 8, **caractérisé en ce que** le ballon du dispositif à traitement medical est calculé et formé pour faciliter l'élimination des obstructions du vaisseau du corps.

11. Dispositif à cathéter de ballon à traitement medical comprenant:

    un cathéter ayant un tube allongé creux définissant un lumen de gonflage s'étendant d'une fin proximale vers une fin distale, et un ballon construit d'un matériel fluoropolymérique biocompatible et se joignant à la fin distale du tube, ledit ballon ayant un mur qui a un épaisseur s'étendant entre une surface intérieure et extérieure et un lumen qui est en communication fluidique avec le lumen de gonflage du cathéter, ledit mur ayant une microstructure des noeuds interconnectés par des fibrilles, ledit ballon est déployable d'un premier état de diamètre réduit à un second état de diamètre agrandi par l'application d'une force d'expansion, **caractérisé en ce que** le mur du ballon inclut au moins une partie à micro pores qui a une porosité suffisante pour laisser un fluide pénétrer le mur où pour l'essentiel tous les noeuds dans la partie à micro pores sont orientés pour l'essentiel perpendiculairement à l'axe longitudinale du ballon.

12. Dispositif à cathéter de ballon à remise du fluide expansible radial ayant un axe longitudinale et un mur perpendiculaire au axe longitudinale où le dispositif à remise du fluide comprend: une première couche du matériel biocompatible ayant une microstructure des noeuds interconnectés par des fibrilles, et une seconde couche du matériel biocompatible ayant une microstructure des noeuds interconnectés par des fibrilles, ladite seconde couche repose la première couche, ledit mur du dispositif à remise du fluide s'étend entre une surface intérieure de la première couche et une surface extérieure de la seconde couche, ledit dispositif à remise du fluide est déployable d'un premier état de diamètre réduit à un second état de diamètre agrandi par l'introduction d'un fluide sous pression, **caractérisé en ce que** le mur du dispositif à remise du fluide inclut au moins une partie à micro pores qui a une porosité suffisante pour laisser un fluide pénétrer le mur où pour l'essentiel tous les noeuds dans la partie à micro pores sont orientés de cette façon que des espaces entre les noeuds forment des microcanaux généralement en ligne orientés et s'étendant de la surface intérieure de la première couche vers la surface extérieure de la seconde couche, lesdits microcanaux sont calculés qu'ils permettent une fluide pénétrer la surface intérieure de la première couche vers la surface extérieure de la seconde couche.

13. Dispositif à remise du fluide selon la revendication 12, **caractérisé en ce que** pour l'essentiel tous les noeuds dans la partie à micro pores sont pour l'essentiel perpendiculaire à l'axe longitudinale de l'élément de construction.

14. Dispositif à remise du fluide selon la revendication 12, **caractérisé en ce que** les microcanaux dans la première couche sont calculés différament que les microcanaux dans la seconde couche.

15. Dispositif à remise du fluide selon la revendication 12, **caractérisé en ce que** les noeuds dans la première couche sont séparés par une première distance internodale et les noeuds dans la seconde couche sont séparés par une seconde distance internodale, **caractérisé en ce que** la première distance internodale est différente de la seconde distance intemodale.

16. Dispositif à remise du fluide selon la revendication 12, **caractérisé en ce que** le matériel biocompatible de la première couche est différente du matériel biocompatible de la seconde couche.

*FIG. 1*

*FIG. 2*

*FIG. 3*

## FIG. 4A

## FIG. 4B

## FIG. 4C

**FIG. 5**

CREATE BILLET — *210*

EXTRUDE BILLET — *212*

REMOVE LUBRICANT FROM EXTRUDATE — *214*

EXPAND EXTRUDATE BY BILATERAL STRETCHING — *216*

HEAT SET EXPANDED TUBE — *218*

POSITION BALLOON WITHIN TUBE — *220*

RADIALLY EXPAND TUBE BEYOND ELASTIC LIMIT BY INFLATION OF BALLOON — *222*

REMOVE BALLOON FROM TUBE — *224*

HEAT SET TUBE — *225*

*FIG. 6A*

FORM ePTFE
TUBE — 410

↓

Position PET
TUBE in ePTFE — 412

↓

Seal end of
PET tube and
connect to inflation
system — 414

↓

Heat and
Radially Expand — 416

↓

Remove PET
tube from
ePTFE tube — 418

↓

HEAT SET
ePTFE Tube — 420

## FIG. 6B

*FIG. 7A*

*FIG. 7B*

*FIG. 7C*

*FIG. 8*

*FIG. 9*

*FIG. 10*

**FIG. 11**

**FIG. 12**

**FIG. 13**

**FIG. 14**

**FIG. 21**

810 — CREATE
BILLET

812 — EXTRUDE
BILLET

814 — REMOVE
LUBRICANT FROM
EXTRUDATE

816 — EXPAND EXTRUDATE
BY BILATERAL
STRETCHING

818 — HEAT SET EXPANDED
TUBE

820 — POSITION BALLOON
WITHIN TUBE

POSITION BALLOON
AND TUBE IN
HEATED CHAMBER — 822

INFLATE
BALLOON — 824

RADIALLY EXPAND
TUBE BEYOND
ELASTIC LIMIT — 826

REMOVE BALLOON
FROM TUBE — 828

HEAT SET
TUBE — 830

ATTACH TUBE
TO DEPLOYMENT
MECHANISM — 832

# FIG. 15

**FIG. 16**

**FIG. 17A**

**FIG. 17B**

FIG. 18

**FIG. 17C**

**FIG. 19**

```
┌─────────────────┐
│   FORM ePTFE    │───── 910
│      TUBE       │
└─────────────────┘
         │
         ▼
┌─────────────────┐
│   Position PET  │───── 912
│  TUBE in ePTFE  │
└─────────────────┘
         │
         ▼
┌─────────────────┐
│   Seal end of   │───── 914
│   PET tube and  │
│ connect to inflation │
│     system      │
└─────────────────┘
         │
         ▼
┌─────────────────┐
│    Heat and     │───── 916
│ Radially Expand │
└─────────────────┘
         │
         ▼
┌─────────────────┐
│   Remove PET    │───── 918
│    tube from    │
│   ePTFE tube    │
└─────────────────┘
         │
         ▼
┌─────────────────┐
│    HEAT SET     │───── 920
│   ePTFE Tube    │
└─────────────────┘
```

# FIG. 20

*FIG. 22*

**FIG. 23A**

**FIG. 23B**

*FIG. 23C*

*FIG. 23D*